# EUROPÄISCHE PATENTSCHRIFT

(11) **EP 1 222 297 B1**
(45) Veröffentlichungstag und Bekanntmachung des Hinweises auf die Patenterteilung: **14.04.2004**
(21) Anmeldenummer: 00979507.1
(22) Anmeldetag: 20.10.2000
(51) Int. Cl.: C12N 15/82, C12N 9/10, C12P 21/00, A01H 5/00, C12P 7/64

(54) **ELONGASEPROMOTOREN FÜR GEWEBESPEZIFISCHE EXPRESSION VON TRANSGENEN IN PFLANZEN**
ELONGASE PROMOTERS FOR THE TISSUE-SPECIFIC EXPRESSION OF TRANSGENES IN PLANTS
PROMOTEURS ELONGASE POUR L'EXPRESSION, SPECIFIQUE D'UN TISSU, DE TRANSGENES DANS DES PLANTES

(30) Priorität: 20.10.1999 DE 19950589
(43) Veröffentlichungstag der Anmeldung: 17.07.2002
(73) Patentinhaber: Gesellschaft für Erwerb und Verwertung von Schutzrechten - GVS mbH, 53115 Bonn (DE)
(72) Erfinder: WOLTER, Frank, P., 53347 Alfter-Oedekoven (DE); HAN, Jixiang, St Louis, MO63141 (US); FRENTZEN, Margrit, 52072 Aachen (DE)
(74) Vertreter: Maiwald Patentanwalts GmbH
(86) Internationale Anmeldenummer: PCT/EP2000/010363
(87) Internationale Veröffentlichungsnummer: WO 2001/029238

(56) Entgegenhaltungen:
- WO-A-01/11061
- WO-A-92/18634
- WO-A-95/07357
- WO-A-95/15387
- WO-A-96/13582
- WO-A-99/49050
- FOURMANN M ET AL: "The two genes homologous to Arabidopsis FAE1 co-segregate with the two loci governing erucic acid content in Brassica napus." THEORETICAL AND APPLIED GENETICS, Bd. 96, Nr. 6-7, Mai 1998 (1998-05), Seiten 852-858, XP000926415 ISSN: 0040-5752
- BARRET P ET AL: "A rapeseed FAE1 gene is linked to the E1 locus associated with variation in the content of erucic acid." THEORETICAL AND APPLIED GENETICS, Bd. 96, Nr. 2, Februar 1998 (1998-02), Seiten 177-186, XP000926412 ISSN: 0040-5752
- ROSCOE TJ ET AL: "Brassica napus fatty acid elongase (FAE1) homolog mRNA" EMBL NUCLEOTIDE SEQU, 6. April 1996 (1996-04-06), XP002109729
- CLEMENS ET AL: "ISOLATION OF A BRASSICA NAPUS CDNA ENCODING 3-" EMBL, XP002163222 -& CLEMENS S. ET AL.: "Isolation of a Brassica napus cDNA (AF009563) encoding 3-ketoacyl-CoA Synthase, a condensing enzyme involved in the biosynthesis of very long chain fatty acids in seeds (PRG 97-125)" PLANT PHYSIOLOGY., Bd. 115, 1997, Seiten 313-314, XP002166114 ROCKVILLE, MD., US ISSN: 0032-0889 in der Anmeldung erwähnt
- DEVIC MARTINE ET AL: "Efficient PCR walking on plant genomic DNA." PLANT PHYSIOLOGY AND BIOCHEMISTRY (PARIS), Bd. 35, Nr. 4, 1997, Seiten 331-339, XP000926417 ISSN: 0981-9428

## Beschreibung

Die vorliegende Erfindung betrifft chimäre Gene mit (i) einer DNA-Sequenz, die für ein gewünschtes Produkt kodiert, und (ii) einem Elongasepromotor, worin die DNA-Sequenz mit dem Promotor operativ verknüpft ist, um Expression des Produkts unter Kontrolle des Promotors zu erlauben. Die Erfindung betrifft weiter Vektoren, Pflanzenzellen, Pflanzen und Pflanzenteile, die das chimäre Gen enthalten sowie Verfahren zur Herstellung solcher Pflanzenzellen, Pflanzen und Pflanzenteile.

Langkettige Fettsäuren, die über 18 Kohlenstoffatome umfassen und auch als very-long-chain fatty acids (VLCFAs) bezeichnet werden, sind in der Natur weit verbreitet. Diese Fettsäuren kommen in erster Linie in Samenölen verschiedener Pflanzenspezies vor, wo sie meist eingebaut in Triacylglyceride vorliegen. In dieser Form findet man VLCFAs insbesondere in Brassicaceae, Tropaeolaceae und Limnanthaceae. Die Samenöle der Brassicaceae-Familie, wie *Brassica napus, Crambe abyssinica, Sinapsis alba, Lunaria annua*, enthalten üblicherweise 40-60% Erucasäure (cis-13-Docosensäure, 22:1^{Δ13}), während in der Tropaeolaceae-Familie auch bis zu 80% Erucasäure im Samenöl vorkommen können. Die Samenöle der *Limnanthes*-Spezies oder Jojoba enthalten sogar mehr als 90% VLCFAs.

In Samenölen akkumulieren VLCFAs gewöhnlicherweise als cis n-9 einfach ungesättigte Fettsäuren wie 20:1^{Δ11}, 22:1^{Δ13} und 24:1^{Δ15}, wobei in einigen Spezies auch VLCFAs vom cis n-7-Typ vorkommen, wie z.B. 20:1^{Δ13} in *Sinapsis alba*, und beispielsweise im Öl von *Limnanthes*-Spezies 20:1^{Δ5} vorherrscht.

Die Anwendungsgebiete pflanzlicher Fette und Öle reichen von Wasch- und Reinigungsmitteln über Kosmetikartikel bis zu Farbzusätzen, Schmierstoffen und Hydraulikölen. Insbesondere ein hoher Gehalt als Erucasäure gilt in der klassischen wie der modernen Pflanzenzüchtung als Zuchtziel, da sie nicht nur als Antischaummittel in Waschmitteln oder als Antiblockiermittel bei der Kunststoffherstellung zum Einsatz kommt, sondern Erucasäure und ihre Derivate, wie Arachinsäure, Pelagonsäure, Brassylsäure und Erucasäureamide, auch als Konservierungsmittel, Aromastoff, Kunststoffweichmacher, Formulierungsmittel, Flotationsmittel, Netzmittel, Emulgator und Gleitmittel Anwendung finden.

VLCFAs werden durch sukzessive Übertragung von C₂-Einheiten von Malonyl-CoA auf langkettige Acylgruppen, die aus der *de novo*-Fettsäuresynthese in the Plastiden stammen. Diese Elongationsreaktionen werden durch Fettsäure-Elongasen (FAE) katalysiert, wobei jeder Elongationszyklus aus vier enzymatischen Schritten besteht: (1) Kondensation von Malonyl-CoA mit einem langkettigen Acylrest, wodurch β-Ketoacyl-CoA entsteht, (2) Reduktion des β-Ketoacyl-CoAs zu β-Hydroxyacyl-CoA, (3) Dehydrierung von β-Hydroxyacyl-CoA to trans-2,3-Enoyl-CoA, (4) Reduktion von trans-2,3-Enoyl-CoA, was in einem verlängerten Acyl-CoA resultiert. Die Kondensationsreaktion, die durch eine β-Ketoacyl-CoA-Synthase (KCS) katalysiert wird, ist der Geschwindigkeits-limitierende Schritt der Kettenverlängerung.

VLCFAs reichern sich vorrangig in Triacylglyceriden von Samen der meisten *Brassica*-Spezies wie *Brassica napus* an. In sich entwickelnden Ölsamen werden Triacylglyceride über den Kennedy-Pathway synthetisiert, an dem in erster Linie die folgenden vier enzymatische Reaktionen beteiligt sind. Zuerst wird Glycerol-3-Phosphat durch Acyl-CoA an der sn-1-Position acyliert, um Lysophosphatidat (sn-1-Acylglycerol-3-Phosphat) zu bilden. Diese Reaktion wird durch eine sn-Glycerol-3-Phosphat-Acyltransferase (GPAT) katalysiert. Dann erfolgt ein zweiter Acylierungsschritt, katalysiert durch eine sn-1-Acylglycerol-3-Phosphat-Acyltransferase (lysophosphatidic acid acyltransferase, LPAAT), wodurch Phosphatidat entsteht, das im nächsten Schritt durch Hydrolyse, katalysiert durch eine Phosphatidat-Phosphatase, zu Diacylglycerol (DAG) umgewandelt wird. Schließlich wird DAG an seiner sn-3-Position durch eine sn-1,2-Diacylglycerol-Acyltraruferase (DAGAT) zu einem Triacylglycerid acyliert.

Während der letzten Jahre konnten KCS-Gene aus *A. thaliana* und Jojoba kloniert werden. Durch Transposon-Tagging mit dem Mais-Transposonaktivator gelang die Klonierung des Fettsäureelongationsgens 1 (FAE1), dessen Produkt an der VLCFA-Synthese beteiligt ist (James *et al*. (1995) Plant Cell 7:309-319). Weiterhin konnte ein cDNA-Klon aus Jojoba von Lassner *et al*. aus einer cDNA-Bank aus sich entwickelnden Samen isoliert werden. (1996, Plant Cell 8:281-292). Kürzlich gelang die Klonierung des *A*. *thaliana* KCS1-Gen (Todd *et al*. (1999) Plant J. 17:119-130). Die Isolierung einer cDNA, die für eine 3-Ketoacyl-CoA-Synthase aus *Brassica napus* kodiert, wurde 1997 von Clemens und Kunst beschrieben (Plant Physiol. 115, 313-314); allerdings scheint die in diesem Stand der Technik offenbarte cDNA-Sequenz nicht für ein aktives Enzym zu kodieren.

Obwohl es sich bei Raps um die wichtigste Produktionsstätte pflanzlicher Öle handelt und die moderne Pflanzenzüchtung aus diesem und weiteren Gründen ein besonders großes Interesse an nützlichen Genen aus eben dieser Kulturpflanze hat, konnte ein β-Ketoacyl-CoA-Synthase-Gen aus Raps, das für ein aktives Enzym kodiert bzw. dessen Übertragung auf transgene Organismen auch tatsächlich in einer nachweisbaren KCS-Aktivität resultiert, bisher nicht erfolgreich isoliert werden.

Rapsöl enthält natürlicherweise hohe Konzentrationen an Erucasäure (∼ 50%) und Rapssorten mit hohem Erucasäuregehalt (high erucic acid rapeseed, HEAR) sind die Hauptquelle für Erucasäure als industrieller Futterbestand. Jedoch ist der gegenwärtige Gehalt von 55% Erucasäure in den Samenölen von HEAR-Sorten nicht ausreichend, um angesichts der hohen Kosten der Aufreinigung der Erucasäure mit den Alternativquellen aus Petrochemikalien konkurrieren zu können. Die Erhöhung des Erucasäuregehalts im Rapsöl mittels gentechnologischer Verfahren könnte hier Abhilfe schaffen und die industrielle Nützlichkeit von Raps als Erucasäureproduzent deutlich verbessern. Andererseits ist Erucasäure aufgrund ihrer unangenehmen geschmacklichen und anderer negativen Eigenschaften als Bestandteil von Lebensmitteln unerwünscht, was während der letzten Jahre zur Züchtung von Rapssorten mit niedrigem Erucasäuregehalt (low erucic acid rapeseed, LEAR) führte, die fast frei von Erucasäure im Samenöl sind. Rapssorten lassen sich somit in für die Industrie interessante HEAR-Sorten einerseits und emährungstechnisch vorteilhafte LEAR-Sorten andererseits unterteilen.

Langkettige Fettsäuren besitzen auch im Nahrungsmittel- und Pharmabereich große Bedeutung. Hier sind es allerdings in erster Linie die mehrfach ungesättigten langkettigen Fettsäuren (engl.: long chain polyunsaturated fatty acids, LC-PUFA), deren essentielle Bedeutung für die menschliche Gesundheit in letzter Zeit immer deutlicher wurde. Es handelt sich um Fettsäuren mit zwei, vor allem aber drei und mehr Doppelbindungen und Kettenlängen von 18 und mehr Kohlenstoffatomen, vor allem aber solchen Kettenlängen von 22 und 24. Wichtige Vertreter sind die Arachidonsäure (5,8,11,14-Eicosatetraensäure), die Eicosapentaensäure (5,8,11,14,17-Icosapentaensäure, EPA) und die Docosapentaensäure (Clupanodonsäure, 4,8,12,15,19-Docosapentaensäure, DHA). Als natürliche Quelle für LC-PUFA steht vor allem Fisch zur Verfügung. Bei dem neuerdings erkannten hohen Bedarf und der jetzt schon bedrohlichen Überfischung der Meere ist der Weltbedarf aus dieser Quelle auf Dauer nicht zu decken. Biotechnologische Methoden zur Herstellung rücken daher in den Vordergrund. Für diese Produktion kommen dabei vor allem Mikroorganismen und Pflanzen in Betracht. Bei den Mikroorganismen bieten sich vor allem Hefen, Pilze und Bakterien an.

Die Biosynthese dieser Fettsäuren verläuft ausgehend von den weit verbreiteten Fettsäuren Linolsäure und alpha-Linolensäure über wechselweise erfolgende Desaturierungs- und Elongationsschritte. Vor allem die hierfür notwendigen Desaturasen sind Objekt intensiver Forschung, deren Gene vor allem aus marinen Mikroorganismen isoliert wurden und dem Fachmann bekannt sind.

Eine Aufgabe der Erfindung besteht in der Bereitstellung eines neuen samenspezifischen Promotors für die Herstellung transgener Pflanzen mit veränderter Genexpression.

Diese Aufgabe wird durch die Isolierung und Charakterisierung eines KCS-Promotors gelöst, der sich für die samenspezifische Expression beliebiger kodierender Regionen in Pflanzen eignet. Wie weiter unten gezeigt werden wird, handelt es sich bei dem KCS-Promotor um einen besonders starken Promotor, der für die gewebespezifische Expression interessanter Gene in Pflanzen besonders nützlich ist. Dabei kann der KCS-Promotor als Translations- oder Transkriptionsfusion mit gewünschten kodierenden Regionen vorliegen und auf Pflanzenzellen übertragen werden. Sowohl die Herstellung geeigneter chimärer Genkonstrukte wie auch die Transformation von Pflanzen mit diesen Konstrukten kann der Fachmann mittels Standardmethoden durchführen. Siehe bspw. Sambrook et al. (1989) Molecular Cloning: A Laboratory Manual, 2. Auflage, Cold Spring Harbor Laboratory Press, Cold Spring Harbor, New York, bzw. Willmitzer L. (1993) Transgenic Plants, in: Biotechnology, A Multi-Volume Comprehensive Treatise (H.J.Rehm, G.Reed, A.Pühler, P.Stadler, eds., Vol.2, 627-659, V.C.H. Weinheim - New York - Basel - Cambridge. Zur Erzeugung der erfindungsgemäßen Pflanzen bieten sich verschiedene Methoden an. Zum einen können Pflanzen bzw. Pflanzenzellen mit Hilfe herkömmlicher gentechnologischer Transformationsmethoden derart verändert werden, daß die neuen Nukleinsäuremoleküle in das pflanzliche Genom integriert werden, d.h. daß stabile Transformanten erzeugt werden. Zum anderen kann ein erfindungsgemäßes Nukleinsäuremolekül, dessen Anwesenheit und gegebenenfalls Expression in der Pflanzenzelle einen veränderten Fettsäuregehalt bewirkt, in der Pflanzenzelle bzw. der Pflanze als selbstreplizierendes System enthalten sein. Zur Vorbereitung der Einführung fremder Gene in höhere Pflanzen stehen eine große Anzahl von Klonierungsvektoren zur Verfügung, deren Replikationssignale für *E*.*coli* und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann in einer passenden Restriktionsschnittstelle in den Vektor eingeführt werden. Das erhaltene Plasmid wird dann für die Transformation von *E*.*coli*-Zellen verwendet. Transformierte *E*.*coli*-Zellen werden in einem geeigneten Medium gezüchtet und anschließend geerntet und lysiert, und das Plasmid wird wiedergewonnen. Als Analysenmethode zur Charakterisierung der gewonnenen Plasmid-DNA werden im allgemeinen Restriktionsanalysen, Gelelektrophoresen und weitere biochemisch-molekularbiologische Methoden eingesetzt. Nach jeder Manipulation kann die Plasmid-DNA gespalten und gewonnene DNA-Fragmente mit anderen DNA-Sequenzen verknüpft werden. Für die Einführung von DNA in eine pflanzliche Wirtszelle stehen eine Vielzahl bekannter Techniken zur Verfügung, wobei der Fachmann die jeweils geeignete Methode ohne Schwierigkeiten ermitteln kann. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, den direkten Gentransfer isolierter DNA in Protoplasten, die Elektroporation von DNA, die Einbringung von DNA mittels der biolistischen Methode sowie weitere Möglichkeiten. Bei der Injektion und Elektroporation von DNA in Pflanzenzellen werden per se keine speziellen Anforderungen an die verwendeten Plasmide gestellt. Ähnliches gilt für den direkten Gentransfer. Es können einfache Plasmide, wie z.B. pUC-Derivate, verwendet werden. Sollen aber aus derartig transformierten Zellen ganze Pflanzen regeneriert werden, ist die Anwesenheit eines selektierbaren Markergens notwendig. Dem Fachmann sind die Genselektionsmarker bekannt, und es stellt für ihn kein Problem dar, einen geeigneten Marker auszuwählen. Je nach Einführungsmethode gewünschter Gene in die Pflanzenzelle können weitere DNA-Sequenzen erforderlich sein. Werden z.B. für die Transformation der Pflanzenzelle das Ti- oder Ri-Plasmid verwendet, so muß mindestens die rechte Begrenzung, häufig jedoch die rechte und linke Begrenzung der im Ti- bzw. Ri-Plasmid enthaltenen T-DNA als Flankenbereich mit den einzuführenden Genen verbunden werden. Werden für die Transformation Agrobakterien verwendet, muß die einzuführende DNA in spezielle Plasmide kloniert werden, und zwar entweder in einen intermediären oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige *vir*-Region. Intermediäre Vektoren können nicht in Agrobakterien replizieren. Mittels eines Helferplasmids kann der intermediäre Vektor auf *Agrobacterium tumefaciens* übertragen werden (Konjugation). Binäre Vektoren können sowohl in *E*. *coli* als auch in Agrobakterien replizieren. Sie enthalten ein Selektionsmarker-Gen und ein Linker oder Polylinker, welche von der rechten und linken T-DNA-Grenzregion eingerahmt werden. Sie können direkt in die Agrobakterien transformiert werden. Das als Wirtszelle dienende Agrobakterium soll ein Plasmid, das eine *vir*-Region trägt, enthalten. Die *vir*-Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet. Die Verwendung von T-DNA für die Transformation von Pflanzenzellen ist intensiv untersucht und ausreichend in allseits bekannten Übersichtsartikeln und Handbüchern zur Pflanzentransformation beschrieben worden. Für den Transfer der DNA in die Pflanzenzelle können Pflanzen-Explantate zweckmäßigerweise mit *Agrobacterium tumefaciens* oder *Agrobacterium rhizogenes* kultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biocide zur Selektion transformierter Zellen enthalten kann, wieder ganze Pflanzen regeneriert werden. Die Regeneration der Pflanzen erfolgt nach üblichen Regnerationsmethoden unter Verwendung bekannter Nährmedien. Die so erhaltenen Pflanzen können dann auf Anwesenheit der eingeführten DNA untersucht werden. Andere Möglichkeiten der Einführung fremder DNA unter Verwendung des biolistischen Verfahrens oder durch Protoplasten-Transformation sind ebenfalls bekannt und vielfach beschrieben. Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biocid oder einem Antibiotikum wie Kanamycin, G418, Bleomycin, Hygromycin, Methotrexat, Glyphosat, Streptomycin, Sulfonyl-Harnstoff, Gentamycin oder Phosphinotricin und u.a. vermittelt. Der individuell gewählte Marker sollte daher die Selektion transformierter Zellen gegenüber Zellen, denen die eingeführte DNA fehlt, gestatten. Die transfomierten Zellen wachsen innerhalb der Pflanze in der üblichen Weise. Die resultierenden Pflanzen können normal angezogen werden und mit Pflanzen, die die gleiche transformierte Erbanlage oder andere Erbanlagen besitzen, gekreuzt werden. Die daraus entstehenden hybriden Individuen haben die entsprechenden phänotyischen Eigenschaften. Von den Pflanzenzellen können Samen gewonnen werden. Es sollten zwei oder mehrere Generationen herangezogen werden, um sicherzustellen, daß das phänotypische Merkmal stabil beibehalten und vererbt wird. Auch sollten Samen geerntet werden, um sicherzustellen, daß der entsprechende Phänotyp oder andere Eigenarten erhalten geblieben sind. Ebenso können nach üblichen Methoden transgene Linien bestimmt werden, die für die neuen Nukleinsäuremoleküle homozygot sind, und ihr phänotypisches Verhalten hinsichtlich eines veränderten Fettsäuregehalts untersucht und mit dem von hemizygoten Linien verglichen werden.

Für die Übertragung eines Resistenzmarkers bietet sich auch eine Co-Transformation an, bei der der Resistenzmarker separat übertragen wird. Die Co-Transferation ermöglicht auf einfache Weise die anschließende Entfernung des Resistenzmarkers durch Auskreuzen.

Gegenstand der Erfindung sind ebenfalls Nukleinsäuremoleküle oder Fragmente davon, die mit einer erfindungsgemäßen Nukleinsäuresequenz oder Promotorregion hybridisieren. Der Begriff "Hybridisierung" bedeutet im Rahmen dieser Erfindung eine Hybridisierung unter konventionellen Hybridisierungsbedingungen, vorzugsweise unter stringenten Bedingungen, wie sie bspw. in Sambrook et al., supra, beschrieben sind. Die mit den erfindungsgemäßen Nukleinsäuresequenzen oder Promotorregionen hybridisierenden Moleküle umfassen auch Fragmente, Derivate und allelische Varianten der Nukleinsäuresequenzen und Promotorregionen. Der Ausdruck "Derivat" bedeutet in diesem Zusammenhang, daß die Sequenzen dieser Moleküle sich von den erfindungsgemäßen Sequenzen an einer oder mehreren Positionen unterscheiden und einen hohen Grad an Homologie zu diesen Sequenzen aufweisen. Homologie bedeutet dabei eine Sequenzidentität von mindestens 70-80% und bevorzugt über 90%. Die Abweichungen können durch Deletion, Addition, Substitution, Insertion oder Rekombination entstanden sein.

Bedingungen, die eine selektive Hybridisierung gewährleisten, kann der Fachmann üblichen Laborhandbüchem entnehmen, beispielsweise Sambrook et al., supra.

Gegebenenfalls können die Nukleinsäuresequenzen oder Promotorregionen der Erfindung durch Enhancer-Sequenzen oder andere regulatorische Sequenzen ergänzt sein. Die regulatorischen Sequenzen beinhalten z.B. auch Signalsequenzen, die für den Transport des Genprodukts zu einem bestimmten Kompartiment sorgen.

Bei den erfindungsgemäßen Pflanzen handelt es sich bevorzugt um Ölsaaten, insbesondere um Raps, Rüpsen, Sonnenblume, Sojabohne, Erdnuß, Kokospalme, Ölpalme, Baumwolle, Lein.

Die Erfindung betrifft auch ein Verfahren zur Bereitstellung samenspezifischer Expression einer kodierenden Region in Pflanzensamen, umfassend die Schritte:
a) Herstellung einer Nukleinsäuresequenz, in der eine Promotorregion, die natürlicherweise upstream einer für ein Protein mit der Aktivität einer KCS kodierenden Sequenz vorliegt, in operativer Verknüpfung mit einer heterologen kodierenden Region vorliegt,
b) Übertragung der Nukleinsäuresequenz aus a) auf pflanzliche Zellen und
c) Regeneration vollständig transformierter Pflanzen und, falls erwünscht, Vermehrung der Pflanzen.

Als kodierende Region, die unter Kontrolle eines erfindungsgemäßen KCS-Promotors in transgenen Pflanzen exprimiert wird, eignet sich jede für ein nützliches Protein kodierende Region, wobei das Protein insbesondere lebensmitteltechnisch, pharmazeutisch oder kosmetisch, landwirtschaftlich oder für die chemische Industrie nützlich sein kann. Beispiele sind Proteine, die eine Rolle in der Fettsäurebiosynthese und im Lipidstoffwechsel spielen, wie Desaturasen und Elongasen, Acyltransferasen, Acyl-CoA-Synthetasen, Acetyl-CoA-Carboxylasen, Thioesterasen. Zu erwähnen sind auch Glykosyltansferasen, Zuckertransferasen und Enyzme, die am Kohlenhydratstoffwechsel beteiligt sind. Prinzipiell aber kann jedes interessante Protein unter Verwendung der erfindungsgemäßen KCS-Promotoren samenspezifisch exprimiert werden, so daß Samen auch allgemein als Bioreaktor für die Expression hochwertiger Proteine genutzt werden können. Die erfindungsgemäßen KCS-Promotoren eignen sich auch allgemein dazu, die Strukturbeschaffenheit und Farbe von Pflanzensamen zu beeinflussen.

Die erfindungsgemäßen Promotorregionen können aber auch vorteilhaft zur gewebespezifischen Ausschaltung unerwünschter Genaktivitäten eingesetzt werden, wobei sich hier Antisense- und Cosuppressionstechniken anbieten.

Die Erfindung betrifft dabei nicht nur chimäre Gene, sondern auch die natürlicherweise vorkommende Kombination des KCS-Promotors mit der für die KCS kodierenden Region.

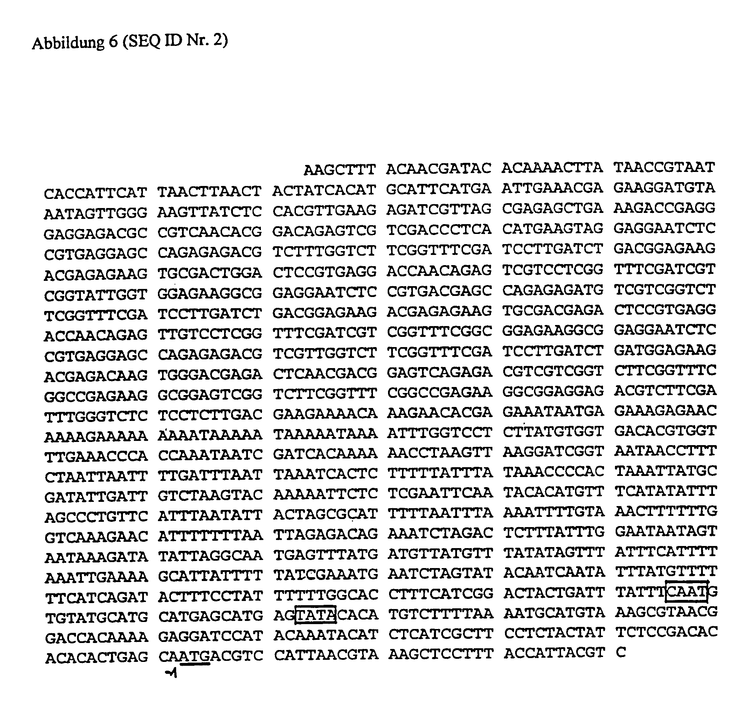

Bei dem KCS-Promotor handelt es sich bevorzugt um eine Promotorregion, die natürlichweise die Expression eines KCS-Gens in Brassicaceen, besonders bevorzugt in *Brassica napus*, kontrolliert. Am meisten bevorzugt handelt es sich bei der Promotorregion um eine Sequenz, die von der in SEQ ID No. 2 dargestellten Sequenz umfasst ist, wobei die Promotorregion mindestens die beiden Promotorelemente TATA-Box und CAAT-Box umfasst (siehe auch Hervorhebung in Abbildung 6).

Die nachfolgenden Beispiele dienen der Erläuterung der Erfindung.

### Beispiele

### Beispiel 1: Isolierung eines vollständigen KCS-cDNA-Klons aus Brassica napus

Ein ca. 1,0 kb langes Fragment wurde mittels PCR unter Verwendung der Primer und aus der kodierenden Region des Arabidopsis-Fettsäureelongationsgens 1 (FAE1, James *et al*., *supra*) amplifiziert. Dieses Fragment wurde als heterologe Sonde für das Screenen einer Raps λ-ZAP cDNA-Library aus unreifen Schoten von *B*. *napus* cv. Askari (Fulda et al. (1997) Plant Mol. Biol. 33:911-922) verwendet. Askari ist eine HEAR-Linie, die in ihrem Samenöl 55% Erucasäure enthält. 5 positive cDNA-Klone konnten aus ca. 1 x 10⁶ Plaques isoliert werden. Restriktionsanalyse ergab, daß alle 5 Klone ein Insert von etwa 1,7 kb Länge enthielten. Sequenzanalyse zeigte, daß die überlappenden Regionen von sowohl dem 5'- als auch dem 3'-Ende der cDNAs identisch waren (etwa 800 bp), daß aber in allen cDNAs 8-14 Nukleotide am 5'-Ende, vermutlich einschließlich des Startcodons, fehlten. Um einen vollständigen cDNA-Klon zu erhalten, wurde eine homologe Sonde von dem längsten cDNA-Klon amplifiziert, unter Verwendung der Oligonukleotidprimer und und für weitere Screeningexperimente mit der cDNA-Library eingesetzt. Da nach zwei weiteren Screeningrunden immer noch kein vollständiger cDNA-Klon gefunden war, wurde eine "nested PCR" mit aus der cDNA-Bank extrahierter Template-DNA eingesetzt, um das 5'-Ende des Inserts zu amplifizieren. Wie die Sequenzanalyse der amplifizierten Fragmente ergab, konnte auch auf diese Weise kein full-length-Klon in der Library detektiert werden. Es wurde daher eine inverse PCR (Ochman et al. (1988) Genetics 120:621-623) für die Klonierung des fehlenden 5'-Endes mit genomischer DNA aus der Askari-Rapslinie als Template eingesetzt. Zwei spezifische Primer und wurden entsprechend dem 5'-Ende der klonierten cDNA, aber in entgegengesetzten Orientierungen konstruiert. Für den Verdau der genomischen DNA wurde das Restriktionsenzym HindIII verwendet, da zwar eine HindIII-Schnittstelle downstream des Primers IP3, aber keine HindIII-Site in der Region zwischen den Primers lag. Nach Verdau und Ligation der genomischen DNA, wurde die Orientierung der Primer umgedreht, so daß die PCR ablaufen konnte. Ein 1,5 kb-Fragment konnte mittels DNA-Polymerasen mit proof reading-Kontrolle, wie z.B. pfu von Stratagene, amplifiziert werden. Das PCR-Fragment wurde kloniert und sequenziert. Die DNA-Sequenzen von drei unabhängigen Klonen waren identisch und enthielten das fehlende 5'-Ende (AGCAATGACGTC, das vermutliche Startcodon ist unterstrichen) der cDNA.

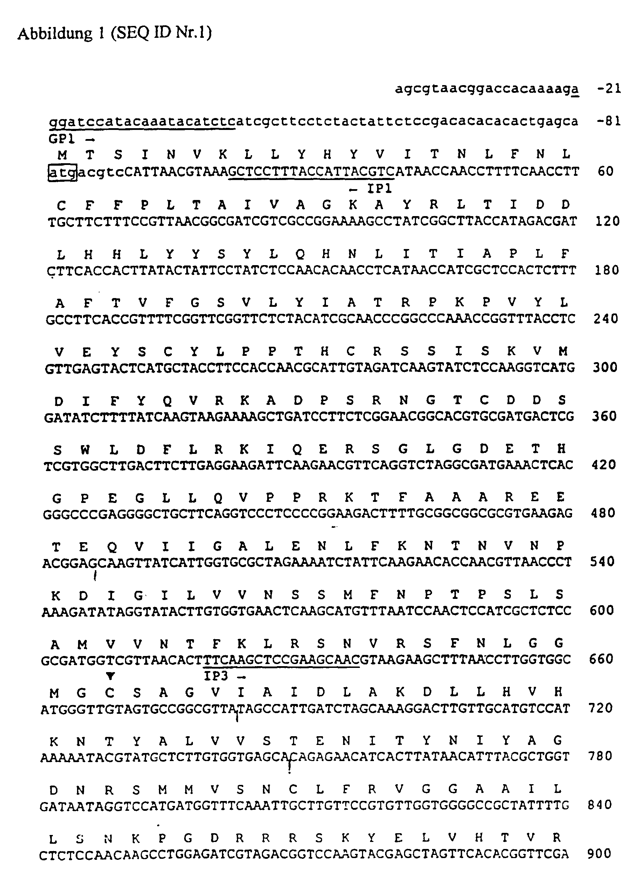

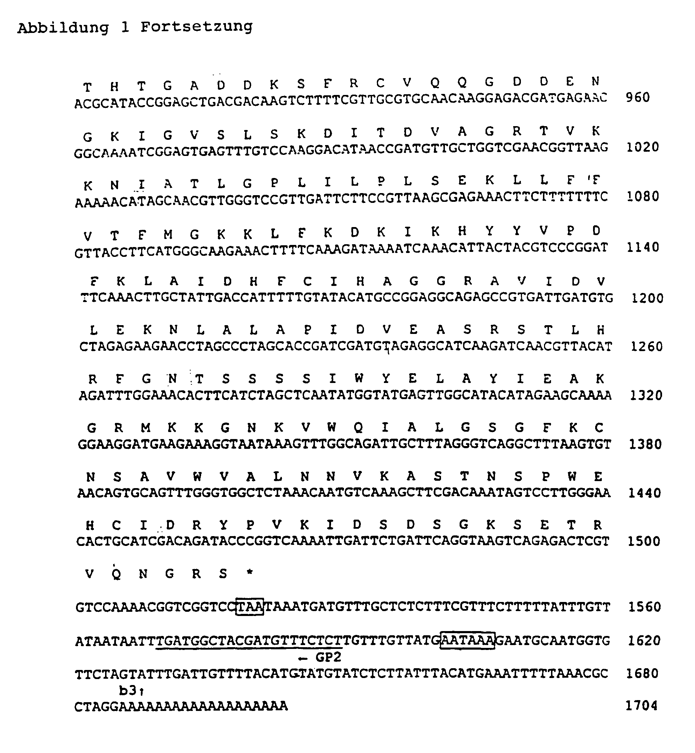

Die vollständige Nukleotidsequenz und die abgeleitete Aminosäuresequenz der KCS-cDNA aus *B*. *napus* cv. Askari ist in Abbildung 1 gezeigt (SEQ ID Nr. 1). Die für die inverse PCR verwendeten Primer sind in Abbildung 1 unterstrichen. Ebenfalls unterstrichen sind die anderen Primer, die für die Amplifikation genomischer DNA aus *B*. *napus* cv. Drakkar und Linie RS306 eingesetzt wurden (siehe Beispiel 2). Forward und reverse Primer sind durch horizontale Pfeile markiert. Das vermutliche Startcodon und Stopcodon sowie die Polyadenylierungssequenz sind umrandet. Das polyA-Signal des Klons #b3 ist durch einen vertikalen Pfeil eingezeichnet. Die vermutliche active site Cys223 ist durch ein gefülltes Dreieck markiert.

Der open reading frame (ORF) hat eine Länge von 1521 bp und kodiert für ein Polypeptid von 506 Aminosäuren (plus Stopcodon), das ein vorhergesagtes Molekulargewicht von 56,4 kDa und einen isoelektrischen Punkt von 9,18 hat.

Für die Analyse des Expressionsmusters des KCS-Gens in *B*. *napus* wurden Northern Blot-Analysen durchgeführt. Zu diesem Zweck wurde Gesamt-RNA aus Blättern und unreifen Embryos von verschiedenen Entwicklungsstadien von Askari-Rapspflanzen nach Standardmethoden isoliert und mit einer *B*. *napus* KCS-cDNA-spezifischen Sonde hybridisiert. Wie erwartet, konnte ein 1,7 kb langes Transkript nur in sich entwickelnden Embryos, aber nicht in Blättern detektiert werden. Dieses Transkript war eindeutig nachweisbar in Embryos 16 Tage nach der Bestäubung, dann nahm seine Konzentration allmählich zu und erreichte ihr Maximum ungefähr 30 Tage nach der Bestäubung, um dann bis zum 40. Tag nach der Bestäubung wieder leicht abzufallen. Diese Northern Blot-Daten zeigten eindeutig, daß die Expression des KCS-Gens sowohl zeitlich als auch räumlich in Wildtyppflanzen von Raps reguliert ist.

### Beispiel 2: Isolierung von genomischen KCS-Klonen aus B. napus

Für die Isolierung genomischer KCS-Klone aus der *B*. *napus*-Linie RS306, einer HEAR-Linie, und aus *B*. *napus* cv. Drakkar, einer LEAR-Sorte (22:1 < 1%), wurden die Primer und abgeleitet von den 5'- und 3'-UTRs der in Abbildung 1 gezeigten cDNA, eingesetzt. Beide genomischen KCS-Sequenzen, aus RS306 und aus Drakkar, enthielten einen ORF von 1521 bp (identisch zu dem ORF der cDNA, siehe Beispiel 1), was bedeutet, daß das KCS-Gen von Raps keine Introns aufweist. Die abgeleiteten Proteine enthalten 506 Aminosäurereste, mit einem Molekulargewicht von 56,46 kDa und einem pI von 9,18 bzw. einem Molekulargewicht von 56,44 kDa und einem pI von 9,23. Verglichen mit der cDNA in Abbildung 1 zeigte die abgeleitete Aminosäuresequenz des genomischen KCS-Klons aus RS306 vier Aminosäureaustausche in den Positionen 286 (Gly286Arg), 323 (Ile323Thr), 395 (Arg395Lys) und 406 (Ala406Gly), während die genomische Sequenz aus Drakkar nur einen Austausch gegenüber der cDNA aus Askari enthielt, nämlich in Position 282 (Ser282Phe).

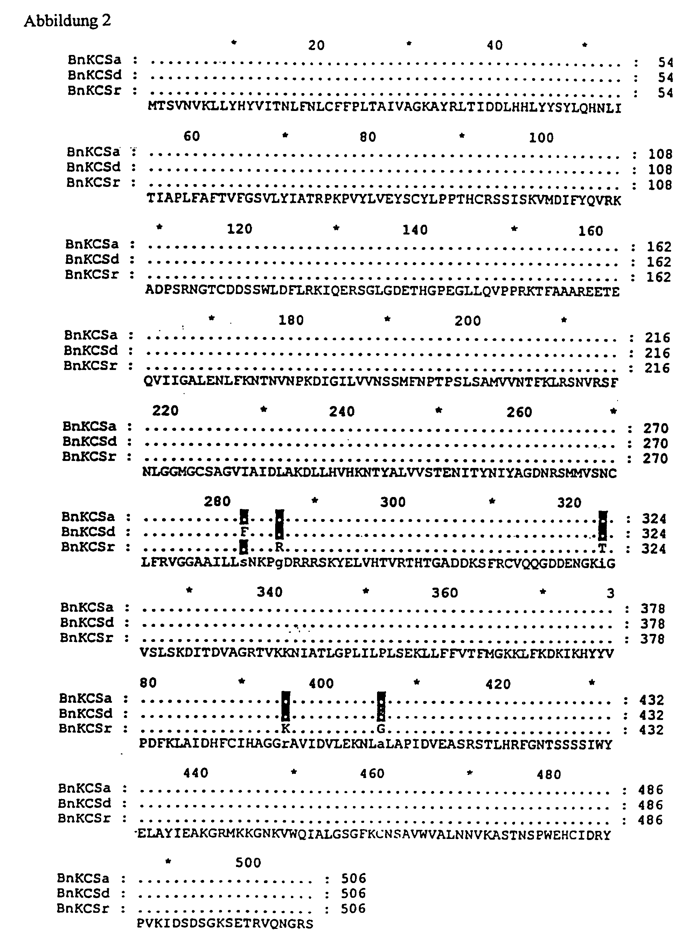

Diese Unterschiede in den Aminosäuresequenzen sind in Abbildung 2 zusätzlich veranschaulicht. BnKCSa = KCS-cDNA aus *B*. *napus* cv. Askari, BnKCSd = genomischer KCS-Klon aus *B*. *napus* cv. Drakkar, und BnKCSr = genomischer KCS-Klon aus *B*. *napus* RS306.

Es wird gegenwärtig angenommen, daß die Mutation in Position 282 (Ser282Phe) in einem katalytisch inaktiven KCS-Protein resultiert und dadurch den LEAR-Phenotyp verursacht.

Verschiedene Hinweise stützen die Hypothese, daß der Rest Ser282 von essentieller Bedeutung für die KCS-Aktivität des Wildtyp-Proteins ist, wobei die Rolle des Serin-Restes eher struktureller als katalytischer Art zu sein scheint.

Schließlich sei darauf hingewiesen, dass sich die in SEQ ID No. 1 dargestellte Sequenz in Aminosäure 307 von der von Clemens und Knust (1997, vide supra) veröffentlichten Sequenz unterscheidet.

### Beispiel 3: Expression von KCS aus B. napus in transgenen B. napus-Pflanzen

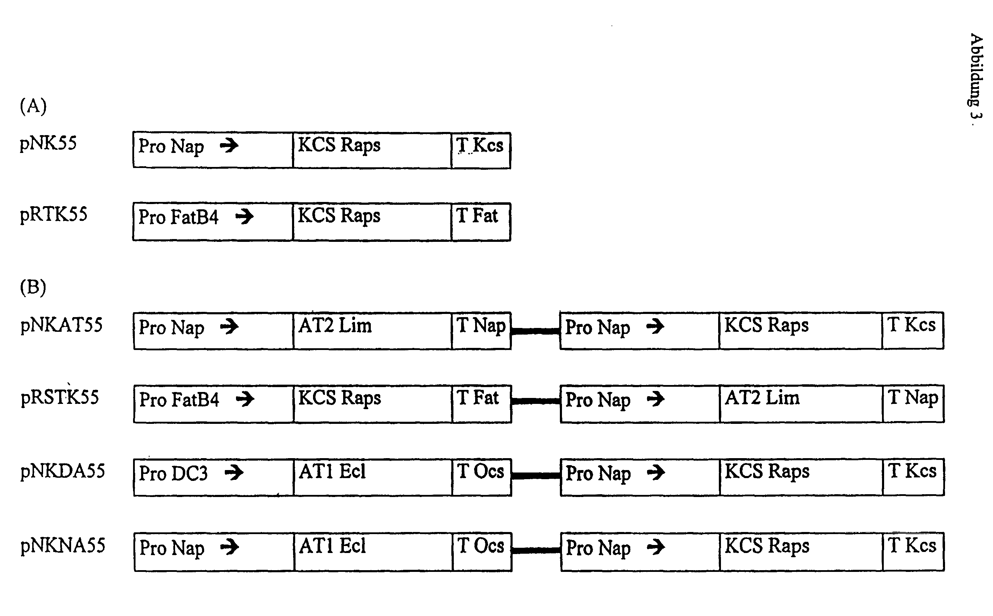

Für die Expression der KCS aus *B*. *napus* cv. Askari in transgenen Pflanzen wurden zunächst verschiedene Plasmidkonstrukte hergestellt, die in Abbildung 3 veranschaulicht sind. Dabei wurde für die Konstruktion von KCS-Gen-Fusionen eine EcoRI-Schnittstelle (unterstrichen in Y1) am 5'-Ende der cDNA durch den Primer eingeführt. Ein 522 bp langes Fragment mit der 509 bp langen kodierenden Region der cDNA und der 13 bp langen 5'-UTR wurde mittels PCR unter Verwendung des Primerpaars Y1/Y2 amplifiziert und im Agarosegel gereinigt; der Primer Y2 hatte die Sequenz

Das Fragment wurde in den pGEM-T-Vektor (Promega) kloniert und sequenziert; der entstandene Vektor wurde mit pNK51 bezeichnet. Die letzten 1,3 kb der cDNA wurden mit ApaI ausgeschnitten und in pNK51, ebenfalls mit ApaI verdaut, ligiert; das entstandene Plasmid wurde pNK52 genannt. Für die Fusion der cDNA mit dem Promotor des Napin-Gens gNA aus *B*. *napus* (Scofield and Crouch (1987) J. Biol. Chem. 262:12202-12208) wurde ein 2,2 kb langes PstI/HindIII-Fragment mit dem Napin-Promotor aus pGEM-Nap ausgeschnitten und in die entsprechenden Schnittstellen des pBluescript KS⁻-Vektors (Stratagene) ligiert; der entstandene Vektor wurde pNK53 genannt. Ein 1,7 kb langes Fragment mit der kodierenden Region der cDNA und ihrem 3'-PolyA-Signal wurde aus pNK52 mit SpeI/BsmI ausgeschnitten und die Enden mit Klenow aufgefüllt. Das resultierende Fragment mit blunt-Enden wurde downstream von dem Napin-Promotor in pNK53, der zuvor mit HindIII verdaut und ebenfalls mit Klenow behandelt worden war, eingeführt, um pNK54 zu ergeben. Ein 3,9 kb-Fragment mit dem chimären KCS-Gen wurde dann in den SpeI/SalI-verdauten binären Vektor pRE1 kloniert, um pNK55 zu liefern. pRE1 weist ein chimäres Neomycin-Phosphotransferase-Gen als Selektionsmarker auf, wobei auch jeder andere für die Transformation von Pflanzen geeignete Vektor und insbesondere jeder andere binäre Vektor eingesetzt werden kann. Für ein Tandemkonstrukt wurde ein 3,3 kb langes SpeI-Fragment, enthaltend ein chimäres *Limnanthes douglasii* LPAAT-Gen, aus pRESS (Weier *et al*. (1997) Fett/Lipid 99:160-165) ausgeschnitten und dann in SpeI-verdauten pNK55 ligiert, wodurch das Konstrukt pNKAT55 entstand.

Für die Konstruktion von Fusionen der für KCS kodierenden Region mit dem Promotor des Acyl-ACP-Thioesterase-Gens FatB₄ aus *Cuphea lanceolata* wurde ein 1,7 kb langes EcoRI/XhoI-BCS-Fragment aus pNK54 zwischen den FatB₄-Promotor und seinem Terminationssignal in einem geeigneten Vektor eingefügt. Ein 5,2 kb-Fragment, enthaltend das chimäre KCS-Gen, wurde mit SfiI ausgeschnitten, seine Enden mit Klenow aufgefüllt und anschließend in pRE1 oder pRESS (Weier *et al*., *supra*), verdaut mit SmaI, kloniert, wodurch die Vektoren pRTK55 bzw. pRSTK55 entstanden.

Für die Herstellung von KCS-Tandemkonstrukten mit einem plsB-Gen, das für die sn-Glycerol-3-Phosphat-Acyltransferase aus *E*. *coli* (Lightner *et al*. (1980) J. Biol. Chem. 19:9413-9420; Lightner *et al*. (1983) J. Biol. Chem. 258:10856-10861) kodiert, wurden zwei Restriktionsschnittstellen, KpnI (unterstrichen in AT1) und MscI (unterstrichen in AT2), mit den zwei Primem bzw. eingeführt. Ein 280 bp langes PCR-Fragment, enthaltend einen samenspezifischen DC3-Promotor aus Karotte (Seffens *et al*. (1990) Dev. Genet. 11:65-76) und eine Leadersequenz Ω aus Tabakmosaikvirus (Gallie *et al*. (1987) Nucl. Acids Res. 15:3257-3273) wurde in pGEM-T (Promega) kloniert, um pGEM-DC3 zu ergeben. Ein 3,0 kb langes HindIII/SmaI-Fragment, enthaltend die 2,5 kb lange plsB-kodierende Region, die 0,25 kb Ocs-Terminationssequenz sowie die 0,25 kb lange 5'-UTR, wurde aus pHAMPL4 (Wolter *et al*. (1992) EMBO J. 11:4685-4692) ausgeschnitten und in HindIII/HincII-verdauten pBluescript KS⁻ kloniert. Die 0,25 kb lange 5'-UTR wurde durch Verdau mit KpnI/MscI entfernt,und ein 300 bp langes DC3Ω-Fragment von pGEM-DC3 wurde dann eingefügt, um pDC3-1AT zu ergeben. Das entstandene chimäre Gen (3,1 kb) wurde in den mit SpeI-verdauten Pflanzenexpressionsvektor pNK55 ligiert, um pNKDA55 zu liefern. Für die plsB-Gen-Fusion mit dem Napin-Promotor wurde ein 2,8 kb langes NcoI/NotI-Fragment, enthaltend die plsB-kodierende Region und den Ocs-Terminator von pDC3-1AT, in den mit den gleichen Enzymen doppelverdauten Vektor pGEM-T (Promega) ligiert. Das entstandene Plasmid pGEM-1AT wurde mit ApaI/NotI geschnitten, mit Klenow behandelt und das Fragment mit blunt-Enden in pNK53, mit HindIII verdaut und mit Klenow behandelt, downstream von dem Napin-Promotor eingefügt. Das entstandene chimäre Gen (5,0 kb) wurde mit SpeI ausgeschnitten und in den ebenfalls mit SpeI verdauten Vektor pNK55 ligiert, um pNKNA55 zu ergeben.

Wie erwähnt, sind die herstellten Pflanzenexpressionskonstrukte in Abbildung 3 schematisch dargestellt; ProNap = Napin-Promotor, ProFatB4 = FatB4-Promotor, ProDC3 = DC3-Promotor, AT2Lim = *Limnanthes* LPAAT-cDNA, KCSRaps = Raps-KCS-cDNA, AT1Ec1 = *E*. *coli* GPAT-Gen, T Kcs, T Fat, T Nap und T Ocs = polyA-Signale von KCS, FatB4, Napin (nap) bzw. *Agrobacterium* Octopin-Synthase (Ocs).

Die erste Gruppe der für die Herstellung transgener Pflanzen eingesetzten Konstrukte besteht somit aus Einzelkonstrukten, in denen die KCS-cDNA unter Kontrolle eines samenspezifischen Promotors, entweder des Napin-Gens gNA aus *B*. *napus* (Scofield *et al*., *supra*) oder des Acyl-ACP-Thioesterase-Gens FatB₄ aus *Cuphea lanceolata*, steht.

Die zweite Gruppe von Konstrukten besteht aus Doppel- oder Tandemkonstrukten, die ein chimäres KCS-Gen in Kombination mit der kodierenden Sequenz entweder der sn-1-Acylglycerol-3-Phosphat-Acyltransferase von *L. douglasii* (LPAAT) (Hanke *et al*. (1995) Eur. J. Biochem. 232:806-810) oder der sn-Glycerol-3-Phosphat-Acyltransferase (GPAT) aus *E. coli*, unter Kontrolle entweder des Napin-Promotors oder des FatB₄-Promotors oder des DC3-Promotors von Karotte (Seffens *et al*., *supra*) plus einer 5'-Leadersequenz (Ω) von Tabakmosaikvirus (Gallie *et al*., *supra*), enthalten (siehe Abbildung 3, B). Diese Konstrukte wurden in geeignete binäre Vektoren eingeführt und für die Rapstransformation auf *Agrobacterium tumefaciens* (Stämme GV3101/pMP90, Koncz and Schell (1986) Mol. Gen. Genet. 204:383-396, und C58ATHV/pEH101, Hood *et al*. (1986) J. Bacteriol. 168:1291-1301) übertragen. Die Einzelkonstrukte wurden auf die LEAR-Sorte Drakkar und die Doppelkonstrukte auf die HEAR-Linie RS306 übertragen.

Die Transformation erfolgte mittels Cokultivierung von Hypokotyl-Explantaten mit transformierten Agrobakterien, und die transgenen Sprosse wurden auf Kanamycin-haltigem Medium nach Standardmethoden selektiert (siehe De Block *et al*. (1989) Plant Physiol. 91:694-701). Transgene Pflanzen wurden mittels Southern Blot-Analyse unter Verwendung geeigneter Sonden auf Anwesenheit der gewünschten Gene überprüft.

Reife Samen von transgenen, selbstbestäubten LEAR-Drakkar-Pflanzen, enthaltend die Napin-KCS- oder FatB₄-KCS-Konstrukte, wurde gesammelt, und gepoolter T2-Samen für die Bestimmung der Fettsäurezusammensetzungen der Samenöle eingesetzt. Die gesammelten Daten sind in der unten stehenden Tabelle 1 zusammengefaßt. Tabelle 1 gibt die Fettsäurezusammensetzung von gepoolten T2-Samen von transgenen LEAR-Drakkar-Pflanzen und von Drakkar-Kontrollpflanzen (ck) wieder. T-NK steht für T2-Samen von Napin-KCS-Pflanzen, während T-RTK T2-Samen von FatB4-KCS-Pflanzen identifiziert.

**Tabelle 1**

| | Gewichtsprozent von Fettsäuren | | | | | | | | |
|---|---|---|---|---|---|---|---|---|---|
| Pflanze | 16:0 | 18:0 | 18:1 | 18:2 | 18:3 | 20:1 | 22:1 | 24:1 | VLCFA |
| Drak (ck) | 3.0 | 1.9 | 66.7 | 15.2 | 8.5 | 1.9 | 0.1 | 0.3 | 2.3 |
| T-NK-4 | 3.1 | 2.2 | 65.1 | 9.8 | 4.6 | 7.3 | 5.6 | 0.4 | 13.3 |
| T-NK-5 | 3.5 | 2.9 | 66.1 | 9.7 | 4.5 | 8.3 | 3.4 | 0.3 | 12.0 |
| T-NK-10 | 3.1 | 2.5 | 65.8 | 9.7 | 4.4 | 8.0 | 4.1 | 0.4 | 12.5 |
| T-NK-11 | 3.5 | 2.4 | 63.9 | 10.2 | 4.6 | 9.3 | 3.9 | 0.4 | 13.6 |
| T-NK-13 | 3.3 | 2.3 | 61.7 | 9.3 | 4.4 | 11.1 | 5.9 | 0.5 | 17.5 |
| T-NK-14 | 3.3 | 2.7 | 69.9 | 11.6 | 4.6 | 4.2 | 1.7 | 0.3 | 6.2 |
| T-NK-15 | 2.9 | 1.9 | 54.7 | 8.6 | 5.5 | 15.1 | 9.1 | 0.5 | 24.7 |
| T-NK-16 | 3.4 | 2.2 | 67.3 | 9.6 | 4.9 | 8.5 | 2.2 | 0.4 | 11.1 |
| T-NK-18 | 3.4 | 2.5 | 67.6 | 9.1 | 4.7 | 8.7 | 2.3 | 0.4 | 11.4 |
| T-NK-20 | 3.1 | 3.5 | 47.2 | 6.6 | 3.5 | 14.8 | 15.5 | 0.7 | 31.0 |
| T-NK-21 | 3.5 | 2.6 | 67.2 | 9.9 | 3.9 | 7.8 | 2.5 | 0.3 | 10.6 |
| T-NK-24 | 3.3 | 2.3 | 73.4 | 9.1 | 4.2 | 4.4 | 1.3 | 0.3 | 6.0 |
| T-NK-26 | 3.1 | 2.3 | 61.8 | 12.5 | 6.7 | 9.0 | 2.1 | 0.2 | 11.3 |
| T-NK-27 | 4.1 | 1.8 | 58.6 | 18.6 | 8.0 | 4.9 | 1.6 | 0.5 | 7.0 |
| T-NK-30 | 2.9 | 1.8 | 58.2 | 11.4 | 6.5 | 12.6 | 4.1 | 0.4 | 17.1 |
| T-NK-32 | 2.9 | 2.1 | 55.0 | 10.9 | 6.6 | 14.2 | 5.6 | 0.5 | 20.3 |
| T-NK-33 | 3.5 | 2.5 | 60.6 | 11.2 | 7.0 | 7.2 | 5.1 | 0.5 | 12.8 |
| T-NK-34 | 3.3 | 1.6 | 60.3 | 15.4 | 8.0 | 7.2 | 1.6 | 0.5 | 9.3 |
| T-NK-35 | 2.6 | 3.2 | 55.4 | 6.2 | 4.1 | 16.4 | 6.7 | 0.6 | 23.7 |
| T-NK-38 | 2.9 | 2.6 | 69.5 | 7.0 | 4.3 | 8.5 | 3.0 | 0.4 | 11.9 |
| T-NK-40 | 3.1 | 1.8 | 65.5 | 11.1 | 7.3 | 6.4 | 2.3 | 0.4 | 9.1 |
| T-NK-41 | 3.2 | 2.7 | 59.6 | 9.7 | 5.7 | 11.7 | 4.8 | 0.5 | 17.0 |
| T-NK-42 | 3.6 | 2.0 | 60.4 | 14.4 | 8.3 | 6.9 | 1.8 | 0.4 | 9.1 |
| T-NK-43 | 3.4 | 1.4 | 59.8 | 14.7 | 10.3 | 7.0 | 1.3 | 0.4 | 8.7 |
| T-NK-47 | 3.2 | 1.8 | 59.9 | 14.7 | 8.7 | 7.6 | 1.6 | 0.4 | 9.6 |
| T-NK-49 | 0.3 | 1.8 | 54.1 | 10.8 | 7.3 | 12.8 | 7.5 | 0.7 | 21.0 |
| T-NK-50 | 2.8 | 2.4 | 64.1 | 9.1 | 5.4 | 9.4 | 2.9 | 0.5 | 12.8 |
| T-NK-65 | 2.9 | 2.2 | 57.1 | 9.5 | 6.0 | 14.6 | 5.4 | 0.5 | 20.5 |
| T-NK-71 | 3.7 | 2.6 | 66.3 | 11.4 | 8.0 | 3.6 | 1.7 | 0.4 | 5.7 |
| T-NK-82 | 3.7 | 2.7 | 61.5 | 10.3 | 5.9 | 11.1 | 4.2 | 0.2 | 15.5 |
| T-NK-85 | 3.9 | 2.3 | 56.8 | 14.9 | 8.6 | 8.8 | 2.7 | 0.4 | 11.9 |
| T-RTK-2 | 3.6 | 2.6 | 67.9 | 10.6 | 4.7 | 7.5 | 1.5 | 0.4 | 9.4 |
| T-RTK-94 | 3.1 | 2.2 | 64.2 | 9.9 | 5.7 | 8.5 | 1.6 | 0.4 | 10.5 |

Das Samenöl der Wildtyppflanzen enthielt weniger als 3% VLCFA, während in der Fettsäurezusammensetzung von transgenen Samenölen bis zu 18% 20:1^{Δ11} und bis zu 16% 20:1^{Δ13} nachweisbar waren. Der Gehalt an 24:1 erreichte in den transgenen Samenölen maximal 0,9%. Während 22 von 44 Napin-KCS-Pflanzen hohe VLCFA-Konzentrationen im Bereich von 11 bis 31% aufwiesen, erreichten nur 2 von 70 FatB4-KCS-Pflanzen einen Gehalt von ungefähr 10% VLCFAs. Allgemein wurde der Anstieg an VLFCAs durch eine Erniedrigung des Gehalts an ungesättigten C 18-Fettsäuren begleitet, während der Gehalt an 16:0 und 18:0 kaum verändert war. Die Unterschiede in den VLCFA-Mengen in den Samenölen unabhängiger Transformanten dürften auf verschiedene KCS-Expressionsraten zurückzuführen sein. Zusammenfassend zeigen die Ergebnisse, daß die cDNA aus *B*. *napus* tatsächlich für eine β-Ketoacyl-CoA-Synthase kodiert, die beide Elongationsschritte von 18:1 zu 22:1 katalysiert, die aber gegenüber 22:1-CoA als Substrat kaum aktiv ist. Die Einführung nur einer KCS als einziges Condensing-Enzym resultiert in signifikanten Mengen an VLCFAs, was bedeutet, daß die drei anderen Enzyme, die für die Synthese von VLCFAs erforderlich sind, nämlich die oben erwähnten zwei Reduktasen und die Dehydratase, im mikrosomalen Elongationssystem von Drakkar-Pflanzen funktionell vorhanden sein müssen.

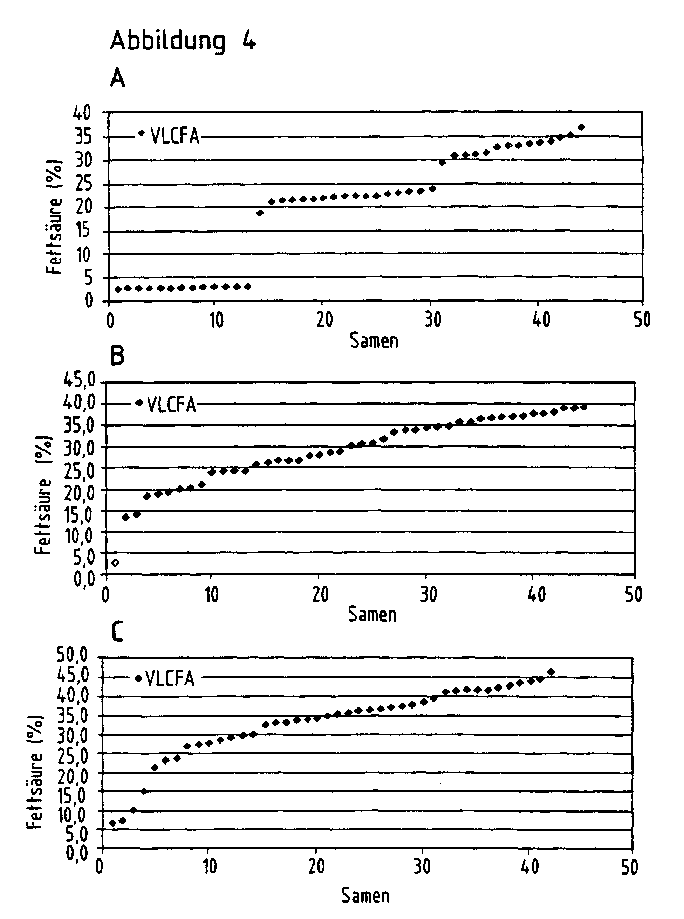

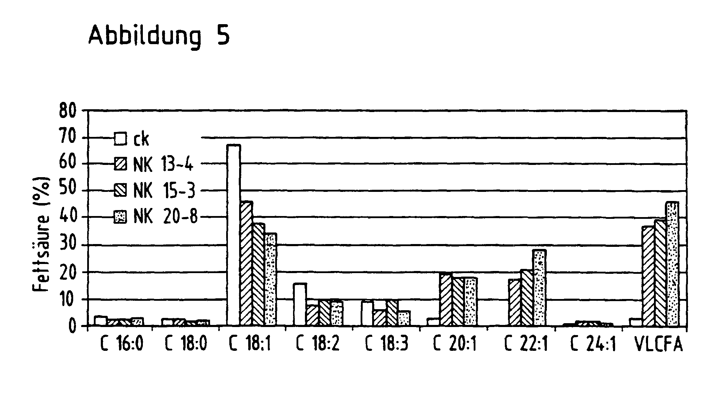

Da T2-Samen für jedes T-DNA-Insert aufspalten, konnte man davon ausgehen, daß einzelne, für das T-DNA-Insert homozygoten Samen einen höheren VLCFA-Gehalt aufweisen. Einzelne Kotyledonen vonT2-Samen von drei transgenen Pflanzen (T-NK-13, -15 und -20) wurden daher für weitere Analysen der Fettsäurezusammensetzung eingesetzt. Die Ergebnisse sind in Abbildung 4 dargestellt, die die Verteilung des VLCFA-Gehalts in einzelnen T2-Samen von transgenen LEAR-Drakkar-Pflanzen zeigt. (A) VLCFA-Gehalt von 44 individuellen Samen von Pflanze T-NK-13, (B) VLCFA-Gehalt von 45 individuellen Samen von Pflanze T-NK-15 und (C) VLDFA-Gehalt von 42 individuellen Samen von Pflanze T-NK-20.Wie erwartet wiesen, verursacht durch Gen-Dosis-Effekte, bestimmte Einzelsamen deutlich höhere VLCFA-Gehalte auf, als die, die in gepoolten Samenölfraktionen gemessen wurden. In T2-Samen der Transformante T-NK-13 zeigten 12 von 44 Samen einen fast zweifach höheren VLCFA-Gehalt als die gepoolten T2-Samen, während 13 Samen das Fettsäuremuster des Wildtyps aufwiesen. Diese Daten zeigen, daß ein T-DNA-Locus in der Primärtransformante von T-NK-13 vorhanden war. Andererseits deutet die Analyse der Transformanten T-NK-15 und T-NK-20 darauf hin, daß diese mindestens drei aktive Kopien des Transgens enthielten, da nur einer von 45 Samen einen in T-NK-15 und kein einziger Samen von 42 in T-NK-20 einen LEAR-Genotyp zeigte. In Einzelsamen von T-NK-20 konnten bis zu 28% 22:1^{Δ13} und 45% VLCFA nachgewiesen werden. Weiter zeigte die Samenölanalyse, daß das Verhältnis von 22:1/20:1 in hohem Maße von der Aktivität des eingeführten KCS-Enzyms abhängig ist, was sich in dem Gesamt-VLCFA-Gehalt in den Samenölen widerspiegelte. 22:1/20: 1-Verhältnisse von > 1 konnten nur bei VLCFA-Gehalten von über 39% beobachtet werden (siehe Abbildung 4 und Abbildung 5). Abbildung 5 zeigt die Fettsäurezusammensetzung von T2-Einzelsamen von transgenen LEAR-Drakkar-Pflanzen, im Vergleich zu Kontrollpflanzen (ck); NK13-4 = Samen einer T-NK-13-Pflanze, NK15-3 = Samen einer T-NK-15-Pflanze, NK20-8 = Samen einer T-NK-20-Pflanze.

Um den Gehalt an Erucasäure in Triacylglyceriden auf der Grundlage von HEAR-Phenotypen zu erhöhen, ist es notwendig, nicht nur den Gehalt an 22:1 im CoA-Samenpool zu erhöhen, sondern den 22:1-Gehalt auch in das Öl und das Sink für Fettablagerung zu kanalisieren. Zu diesem Zweck wurden die oben beschriebenen Expressionsvektoren konstruiert, in denen die Raps-KCS in Kombination mit entweder LPAAT (von *L*. *douglasii*) mit dem Ziel der Manipulation der Kanalisierung von 22:1 in die sn-2-Position des Samenöls oder GPAT (von *E*. *coli*) mit dem Ziel der Erhöhung der Sinkkapazität für Fettablagerung, unter der Kontrolle von entweder dem Napin-Promotor oder dem FatB4-Promotor oder dem DC3-Promotor, vorliegt. Die Konstrukte, NKAT (napin-KCS-napin-LPAAT), RSTK (FatB4-KCS-napin-LPAAT), NKDA (napin-KCS-DC3-GPAT= und NKNA (napin-KCS-napin-GPAT) wurden auf die HEAR-Linie RS306 übertragen. Gepoolte T2-Samen von transgenen RS306-Pflanzen wurden hinsichtlich ihrer Fettsäurezusammensetzung analysiert, die Ergebnisse sind in Tabelle 2 zusammengefaßt. RS306 (ck) identifiziert das Samenöl von RS306-Kontrollpflanzen, die mit dem leeren Vektor pRE1 transformiert wurden. T-NKAT repräsentiert T2-Samen von NKAT-Pflanzen, T-RSTK T2-Samen von RSTK-Pflanzen, T-NKDA T2-Zellen von NKDA-Pflanzen und T-NKNA T2-Samen von NKAT-Pflanzen.

**Tabelle 2**

| | Gewichtsprozent von Gesamtfettsäuren | | | | | | | | TAGSpezies | |
|---|---|---|---|---|---|---|---|---|---|---|
| Pflanze | C16:0 | C18:0 | C18:1 | C18:2 | C18:3 | C20:1 | C22:1 | C24:1 | EiEE | EEE |
| RS306 (ck) | 2.5 | 1.3 | 15.7 | 10.8 | 4.1 | 6.5 | 53.7 | 1.9 | - | - |
| T-NKAT-1 | 2.4 | 1.1 | 13.3 | 11.5 | 5.1 | 7.0 | 55.0 | 1.6 | 2.8 | 2.9 |
| T-NKAT-5 | 2.3 | 1.3 | 13.0 | 10.1 | 4.1 | 6.3 | 56.7 | 1.6 | 3.0 | 3.7 |
| T-NKAT-6 | 2.1 | 1.0 | 11.8 | 10.4 | 5.3 | 8.2 | 55.5 | 1.5 | 4.3 | 4.1 |
| T-NKAT-7 | 2.0 | 0.9 | 12.7 | 10.8 | 4.4 | 8.3 | 55.3 | 1.5 | 4.2 | 4.1 |
| T-NKAT-14 | 2.1 | 0.9 | 11.9 | 11.6 | 5.4 | 6.1 | 55.9 | 1.7 | 3.8 | 4.3 |
| T-RSTK-13 | 2.1 | 0.8 | 11.1 | 11.1 | 6.4 | 5.9 | 56.7 | 1.9 | 4.3 | 5.6 |
| T-RSTK-15 | 2.0 | 1.0 | 14.5 | 10.7 | 4.1 | 7.0 | 55.3 | 1.6 | 3.5 | 2.9 |
| T-NKDA-5 | 1.9 | 1.2 | 12.1 | 11.0 | 4.9 | 6.4 | 58.2 | 2.0 | - | - |
| T-NKDA-7 | 2.3 | 1.2 | 11.4 | 10.0 | 5.1 | 5.2 | 59.6 | 2.3 | - | - |
| T-NKDA-15 | 1.9 | 1.2 | 11.1 | 11.2 | 4.5 | 5.8 | 58.7 | 2.1 | - | - |
| T-NKDA-16 | 1.8 | 1.2 | 12.5 | 11.3 | 4.9 | 5.3 | 58.0 | 1.9 | - | - |
| T-NKDA-9 | 2.1 | 1.4 | 11.7 | 11.2 | 4.5 | 5.7 | 57.0 | 2.7 | - | - |
| T-NKDA-4 | 1.9 | 0.9 | 10.0 | 13.5 | 5.9 | 5.1 | 57.6 | 1.8 | - | - |
| T-NKNA-3 | 1.6 | 1.0 | 12.8 | 11.0 | 5.0 | 5.4 | 58.7 | 2.0 | - | - |
| T-NKNA-15 | 2.0 | 1.3 | 10.7 | 12.4 | 5.4 | 4.8 | 56.3 | 2.4 | - | - |
| T-NKNA-20 | 1.8 | 1.1 | 16.1 | 8.7 | 4.3 | 8.1 | 56.4 | 1.7 | - | - |

In Tabelle 2 steht "EiEE" für Triacylglycerid mit einem Eicosensäure-Rest (20:1) und zwei Erucasäure-Resten (22:1). "EEE" steht für Trierucin, also Triacylglycerid mit drei Erucasäuren.

In T2-Samenölen konnte ein leichter Anstieg im 22:1-Gehalt beobachtet werden, der im Bereich von 2,6 bis 5,9%, verglichen mit dem von RS306-Kontrollpflanzen, lag. Die transgenen Pflanzen akkumulierten 2,9-5,6% Trierucin (EEE) in ihrem Samenöl. Der Anteil von 22:1 an der sn-2-Position im Triacylglycerid (TAG) erreichte 31,7-37,5% in den transgenen Samenölen, während der Anteil in den Kontrollsamen weniger als 1 % der sn-2-Fettsäuren ausmachte. Diese Ergebnisse zeigten, daß die eingeführten KCS- und LPAAT-Gene in den transgenen Pflanzen funktionell exprimiert wurden. Weiter lassen die in Tabelle 2 gezeigten Daten vermuten, daß maximal ein Gehalt von 22:1 von 60-65% in HEAR-Pflanzen erreicht werden kann.

### Beispiel 4: Analyse des Raps-KCS-Promotors

Im Rahmen der Vervollständigung des Bereichs des Startkodon der KCS-cDNA wurde, wie oben in Beispiel 1 beschrieben, eine inverse PCR durchgeführt und verschiedene 5'-flankierende Sequenzen der KCS-kodierenden Region mit einer Länge von - 1,5 kb aus der genomischen DNA von drei verschiedenen Rapssorten (*B*. *napus* cv. Askari, Drakkar und RS-Linie 306) isoliert. Sequenzanalysen zeigten, daß die Promotorsequenzen dieser Klone identisch waren, weshalb ein aus Askari isolierter Promotor für die weitere Analyse ausgewählt wurde.

Abbildung 6 zeigt die Sequenz des KCS-Promotors aus Raps (SEQ ID Nr. 2); die Sequenz umfaßt insgesamt 1468 Basen. Das 5'-Ende der gezeigten Sequenz entspricht dem Nukleotid -1429 des KCS-Gens, am 3'-Ende umfaßt die gezeigte Sequenz die Kodons 1 (Methionin) bis 13 (Valin) der KCS-kodierenden Sequenz. Das ATG-Startcodon, die CAAT-Box sowie die TATA-Box sind eingezeichnet.

Ähnlichkeiten der KCS-Promotorregion mit irgendwelchen anderen Promotorsequenzen, die in den Datenbanken erhältlich sind, wurden nicht beobachtet.

Der KCS-Promotor zeigt nicht nur die für samenspezifische Promotoren typischen AT-reichen Elemente (19 Elemente mit einer Länge zwischen 6 und 19 bp im Bereich zwischen - 1 und -471), sondern auch verschiedene andere Motive im Bereich -99 bis -137, die eine gewebespezifische Regulation vermuten lassen. Ein RY-Repeat (CATGCATG) liegt zwischen der CAAT-Box und der TATA-Box, und eine E-Box (CACATG) befindet sich direkt neben der TATA-Box.

Für die Analyse der funktionellen und gewebespezifischen Expression in transgenen Rapspflanzen wurde die 1,5 kb lange Promotorregion des KCS-Gens mit dem für β-Glucuronidase (GUS) kodierenden Reportergen uidA (Jefferson *et al*. (1987) Plant Mol. Biol. Rep. 5:387-405; Jefferson *et al*. (1989) EMBO J. 6:3901-3907) in dem binären Vektor pBI101.2 (Clontech, CA; Jefferson *et al*., *supra*) fusioniert. Zu diesem Zweck wurde zunächst eine PCR unter Verwendung folgender Primer durchgeführt: und wobei IP6 mit der Promotorregion überlappt und der Reverseprimer IP8 eine eingeführte SmaI-Schnittstelle (unterstrichen) für die Klonierung enthielt. Ein 470 bp PCR-Fragment wurde in den Vektor pGEM-T (Promega) ligiert und sequenziert. Das PCR-Fragment wurde mit den Restriktionsenzymen EcoRI und NcoI ausgeschnitten und in das 3'-Ende des mit den gleichen Enzymen geschnittenen Promotors ligiert. Schließlich wurde ein 1,5 kb-Promotorfragment mit den Restriktionsenzymen HindIII und SmaI ausgeschnitten und vor die GUS-kodierende Region im pBI101.2 eingefügt. Das hieraus entstandene Konstrukt wurde mit pBnKCS-Prom bezeichnet.

Das Promotor/GUS-Konstrukt wurde auf *B*. *napus* RS306 übertragen, und unreife Samen in verschiedenen Entwicklungsstadien und andere Gewebe von transgenen Pflanzen wie Kontrollpflanzen wurden für die GUS-Analyse eingesetzt. Die histochemische GUS-Anfärbung zeigte GUS-Aktivität nur in sich entwickelnden Samen von transgenen Pflanzen, aber nicht in Wurzeln, Stengeln, Blättern, Knospen und Blüten und auch nicht in Organen der Kontrollpflanzen. In transgenen Samen war GUS-Expression erstmals am 16. Tag nach der Bestäubung im histochemischen Test sichbar und wurde bis zum 30. Tag nach Bestäubung stärker, was dem Expressionsmuster des nativen KCS-Gens entspricht. Die quantitative Analyse mittels Chemilumineszenz. bestätigte die histochemischen Ergebnisse. In transgenem Samen, der am 25. und 30. Tag nach Bestäubung geerntet wurde, konnten GUS-Aktivitäten von bis zu 180 bzw. 324 µmol/min/mg Protein gemessen werden. Diese Daten beweisen, daß die in Abbildung 6 gezeigte Promotorregion einen neuen, sehr aktiven samenspezifischen Promotor mit hoher Expressionsrate in transgenen Rapspflanzen darstellt.

### Beispiel 5: Expression von KCS aus B. napus in Hefe

Um die Funktion und Aktivität der durch die verschiedenen isolierten KCS-Gene aus Askari, Drakkar und der RS-Linie 306 kodierten KCS vergleichen zu können, wurden die Gene in den *Saccharomyces cerevisiae*-Stamm INVSC1 (Invitrogen) unter der Kontrolle eines Galactose-induzierbaren GAL1-Promotors exprimiert.

Zu diesem Zweck wurden die verschiedenen isolierten KCS-Sequenzen in dem Hefeexpressionsvektor pYES2 (Invitrogen, CA) mit dem GAL1-Promotor fusioniert. Ein 1,7 kb BnKCSa-Fragment aus der cDNA-Bibliothek von *B*. *napus* cv. Askari wurde mit den Restriktionsenzymen EcoRI und XhoI ausgeschnitten und in mit denselben Enzymen geschnittenen Vektor pYES2 eingefügt, wodurch der Vektor pYES-BnKCSa entstand. Für die anderen beiden Hefeexpressionskonstrukte wurde ein 0,8 kb HindIII-Fragment von BnKCSa durch das von BnKCSd, also die genomische DNA-Sequenz von *B*. *napus* cv. Drakkar ersetzt. Das resultierende 1,7 kb chimäre BnKCSd-Gen wurde in EcoRI/XhoI-verdauten Vektor pYES2 eingefügt, wodurch der Vektor pYES-BnKCSd entstand. Für das letzte Konstrukt, nämlich den Hefeexpressionsvektor enthaltend die genomische KCS-Sequenz der Linie RS306 wurde ein 0,9 kb ClaI/EcoRV-Fragment von BnKCSa durch das von BnKCSr (KCS-Sequenz aus der Linie RS306) ersetzt. Die Plasmid-DNAs wurden aus dem *E*. *coli*-Stamm SCS110 (Stratagene) isoliert. Das entstandene chimäre BnKCSr-Gen (1,7 kb) wurde in EcoRI/XhoI-verdauten pYES2 eingefügt, um pYES-BnKCSr zu ergeben.

INVSC1-Zellen mit dem Plasmid pYES2 ohne Insert wurden als Wildtyp-Kontrolle verwendet. Die Fettsäurezusammensetzung der Hefezellen wurde mittels Gas-Flüssig-Chromatographie (GLC) bestimmt, und die Bestandteile von VLCFAs wurden weiter mittels GLC-MS-Analyse (GLC-Massenspektrometrie) identifiziert. Beträchtliche Mengen an VLCFAs wurden in den transgenen Hefezellen mit der KCS-Sequenz aus Askari gefunden, während die transgenen Hefezellen, die die KCS-Sequenzen aus Drakkar bzw. der RS-Linie exprimierten, Fettsäurezusammensetzungen zeigten ähnlich den Kontrollzellen (siehe auch Tabelle 3). In Zellen mit der KCS-Sequenz aus Askari wurden bis zu 41% VLCFAs in den Fettextrakten detektiert, in denen 22:1-Fettsäuren mit Doppelbindung in entweder der Position Δ15 oder Δ13 vorherrschten, aber auch gesättigte und einfach ungesättigte Fettsäuren mit mehr als 22 C-Atomen ebenfalls in nennenswerten Mengen detektiert werden konnten. Diese Daten zeigen, daß das KCS-Gen von Askari, anders als die KCS-Gene von Drakkar oder der RS306-Linie, in Hefe funktionell exprimiert wurde und mit den Bestandteilen des Hefe-Elongase-Komplexes effektiv zusammenwirkte. Weiter zeigen die Daten, daß diese in Hefe exprimierte KCS eine relativ breite Acyl-CoA-Spezifität besitzt.

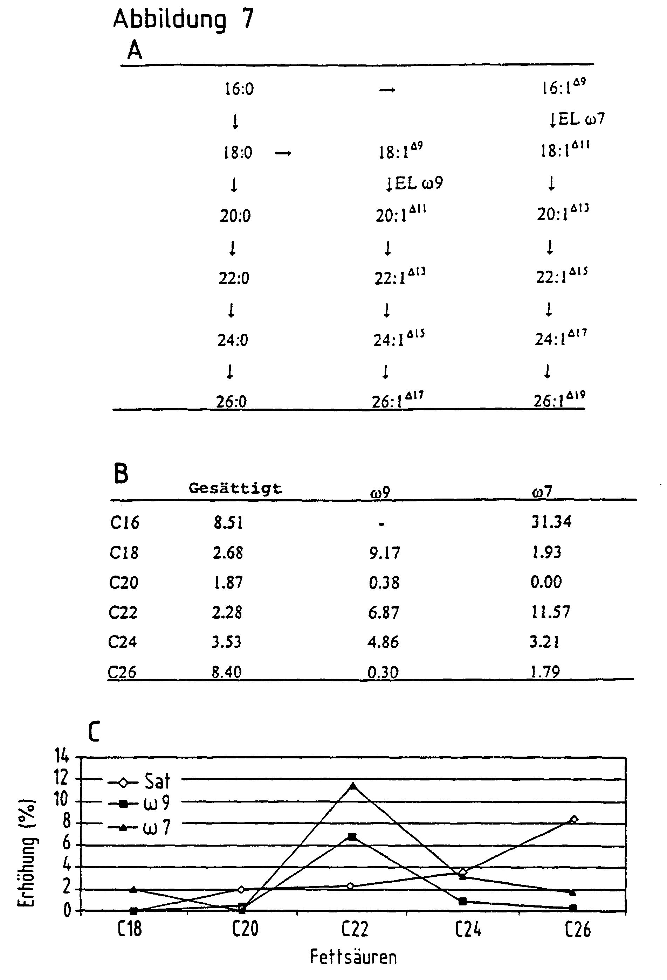

Wie in Abbildung 7A dargestellt, verwendet die KCS nicht nur 18:1^{Δ9}-, sondern auch 16:1^{Δ9}-Acylgruppen als Substrat. Da Hefezellen zweimal mehr 16:1^{Δ9} als 18:1^{Δ9} akkumulieren, scheint die KCS beide Acylgruppen in ähnlichem Ausmaß zu verwenden. Zusätzlich zeigte die Fettsäureanalyse von transgenen Hefezellen, daß die eingeführte KCS aus Askari die Elongation von 18:0 derart bewirkt, daß 26:0 als Hauptprodukt gebildet wird. Somit scheint die Fähigkeit der Askari-KCS, C20- und C22-Acylgruppen zu verlängern, deutlich höher mit gesättigten als mit einfach ungesättigten Acyl-CoA-Thioestern zu sein. Insgesamt zeigen die Daten, daß die KCS aus Askari äußerst aktiv in Hefe ist und zudem in der Lage ist, vier bis fünf Elongationsschritte in Hefe zu katalysieren. Hier scheint die KCS aus *Brassica napus* der KCS aus A. *thaliana*, die nur zwei bis drei Elongationsschritte katalysiert, deutlich überlegen zu sein.

Wie oben erwähnt, konnte kein VLCFA-Gehalt in Hefezellen, transformiert mit KCS aus Drakkar, detektiert werden. Wie bereits erwähnt, zeigen die abgeleiteten Aminosäure-Sequenzen nur einen Unterschied in Position 282, nämlich daß Serin in Drakkar in dieser Position durch Phenylalanin ersetzt ist. Dieser Aminosäureaustausch könnte ein katalytisch inaktives Protein hervorrufen und dadurch den LEAR-Phenotyp der Sorte Drakkar bewirken. Dies wird auch durch Daten der Analyse des Samenöls von transgenen Drakkar-Pflanzen bestätigt, die zeigen, daß der Phenotyp mit höherem Erucasäuregehalt durch Einführung des KCS-Gens aus Askari wiederhergestellt werden kann.

Die untenstehende Tabelle 3 zeigt die Fettsäurezusammensetzung von Wildtyp-Kontroll- und transformierten Hefezellen. YES2 = Wildtyp-Kontrolle; BnKCSa = Hefezellen, transformiert mit Askari BnKCS; BnKCSd = Hefezellen, transformiert mit Drakkar BnKCS; BnKCSr = Hefezellen, transformiert mit RS306 BnKCS. Die Werte geben den Gehalt an einer bestimmten Fettsäure als prozentualen Anteil (w/w) des Gesamtfettsäuregehalts wieder.

**Tabelle 3**

| Fettsäure | YES2 | BnKCSa | BnKCSd | BnKCSr |
|---|---|---|---|---|
| 16:0 | 22.83 | 8.51 | 23.78 | 23.08 |
| 16:1^{Δ9} | 45.79 | 31.34 | 44.90 | 44.27 |
| 18:0 | 6.20 | 2.68 | 7.06 | 6.61 |
| 18:1^{Δ9} | 24.00 | 9.17 | 22.97 | 24.55 |
| 18:1^{Δ11} | - | 1.93 | - | - |
| 20:0 | - | 1.87 | - | - |
| 20:1^{Δ11} | - | 0.38 | - | - |
| 22:0 | - | 2.28 | - | - |
| 22:1^{Δ13} | - | 6.87 | - | - |
| 22:1^{Δ15} | - | 11.57 | - | - |
| 24:0 | - | 3.53 | - | - |
| 24:1^{Δ15} | - | 0.86 | - | - |
| 24:1^{Δ17} | - | 3.21 | - | - |
| 26:0 | - | 8.40 | - | - |
| 26:1^{Δ17} | - | 0.30 | - | - |
| 26:1^{Δ19} | - | 1.79 | - | - |

Die nachstehende Abbildung 7 enthält Daten der Expression von BnKCSa in Hefe, wobei (A) verschiedene Synthesewege für verschiedene VLCFAs zeigt; (B) die Fettsäurezusammensetzungen von Hefezellen, transformiert mit BnKCSa, zeigt, und (C) die erhöhten Anteile verschiedener VLCFA-Spezies am Gesamtfettsäuregehalt wiedergibt.

Lipidextraktionen und Fettsäureanalysen wurden nach Standardverfahren durchgeführt, siehe z.B. Browse et al. (1986) Anal. Biochem. 152: 141-145, wobei die Fettsäuremethylester weiter durch GC-MS-Analyse unter Verwendung ihrer Nikotinat- und Di-O-Trimethylsilylether-Derivate identifiziert wurden (Dommes et al. (1976) J. Chromatogr. Sci. 14: 360-366; Murata et al. (1978) J. Lipid Res. 19: 172-176).

### Beispiel 6: Fettsäurefütterungsexperimente mit transgenen Hefezellen, die die KCS aus B. napus exprimieren

Um die Substratspezifitäten der in Hefezellen exprimierten KCS aus *B*. *napus* zu analysieren, wurden Fütterungsversuche mit verschiedenen, mehrfach ungesättigten Fettsäuren durchgeführt. Für diese Versuche wurden die transgenen Hefezellen, wie in Beispiel 5 beschrieben, entwickelt und kultiviert. Bei einer optischen Dichte der Hefekulturen von 0,5 wurde die Genexpression durch Zugabe von 2% Galactose induziert. Zu diesem Zeitpunkt wurden die Kulturen ebenfalls in Gegenwart von 0,1% Tergitol NP-40 mit verschiedenen Fettsäuren in einer Endkonzentration von 0,2 mM versetzt und 24 h bei 30°C weiter kultiviert (Kontrolle ohne Fettsäurezusatz). Schließlich wurden die Zellen geerntet und für die Fettsäureanalyse verwendet.

Durch Kontrollexperimente wurde sichergestellt, daß Hefezellen selbst nicht in der Lage sind, die eingesetzten Substrate 18:2^{9,12}, 18:3^{9,12,15}, 18:3^{6,9,12}, 20:3^{8,11,14} und 20:4^{5,8,11,14} zu elongieren. Überraschenderweise wurden in solchen Hefezellen, in denen die KCS aus *B*. *napus* exprimiert wurde, je nach eingesetztem Substrat verschiedene Elongationsprodukte gefunden, die in Tabelle 4 unten aufgeführt sind. Diese gefundenen Elongationsprodukte sind auf die Aktivität der eingeführten KCS aus *B*. *napus* zurückzuführen.

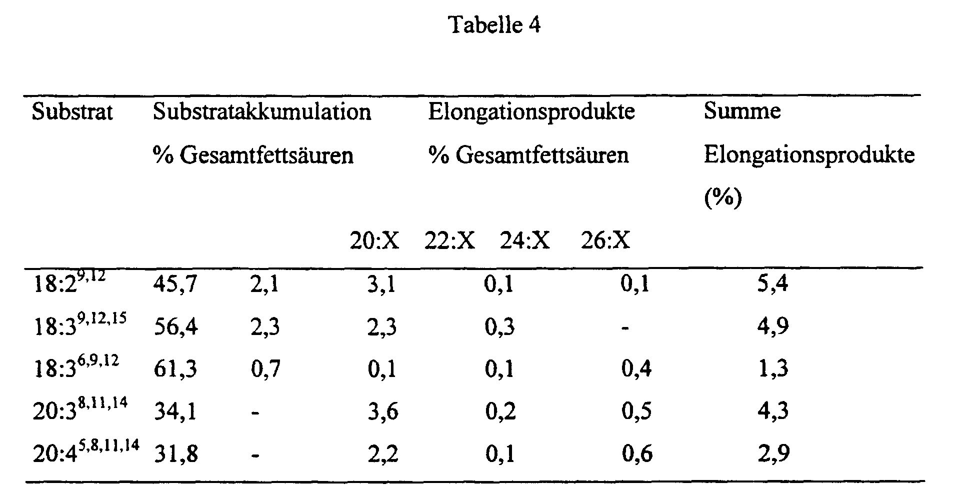

Aus den in Tabelle 4 zusammengefaßten Daten wird deutlich, daß die in den Hefezellen exprimierte KCS aus *B*. *napus* (BnKCSa) die exogen zugesetzten Fettsäuren, 18:2^{9,12}, 18:3^{9,12,15}, 18.3^{6,9,12}, 20:3^{8,11,14}, 20:4^{5,8,11,14}, die von den Hefezellen aus dem Medium aufgenommen wurden, als Substrate nutzt und um 6 bis 8 C-Atome verlängert. Die mit 20:X, 22:X, 24:X und 26:X bezeichneten Produkte entsprechen den erwarteten Elongationsprodukten. Die korrekte Lage der Doppelbindungen wurde durch GC/MS sichergestellt.

## Patentansprüche

1. Isolierte Promotorregion, die natürlicherweise die Expression eines pflanzlichen β-Ketoacyl-CoA-Synthase-Gens kontrolliert und eine Nukleotidsequenz aufweist, die mindestens die beiden Promotorelemente TATA-Box und CAAT-Box umfasst, wobei diese Nukleotidsequenz entweder von der in SEQ ID No. 2 dargestellten Sequenz umfasst ist, oder mit der in SEQ ID No. 2 dargestellten Promotorregion unter stringenten Hybridisierungsbedingungen hybridisiert, oder mit der in SEQ ID No. 2 dargestellten Promotorregion eine Sequenzidentität von mindestens 70% - 80% aufweist.

2. Die Promotorregion gemäß Anspruch 1, die eine Nukleotidsequenz aufweist, die von der in SEQ ID No. 2 dargestellten Sequenz umfasst ist und mindestens die beiden Promotorelemente TATA-Box und CAAT-Box umfasst, und mit der in SEQ ID No. 2 dargestellten Promotorregion unter stringenten Hybridisierungsbedingungen hybridisiert.

3. Die Promotorregion gemäß Anspruch 1, die eine Nukleotidsequenz aufweist, die von der in SEQ ID No. 2 dargestellten Sequenz umfasst ist und mindestens die beiden Promotorelemente TATA-Box und CAAT-Box umfasst, und mit der in SEQ ID No. 2 dargestellten Promotorregion eine Sequenzidentität von mindestens 70 % - 80 % aufweist.

4. Die Promotorregion gemäß Anspruch 1, die eine Nukleotidsequenz aufweist, die mit der in SEQ ID No. 2 dargestellten Promotorregion unter stringenten Hybridisierungsbedingungen hybridisiert, und mit der in SEQ ID No. 2 dargestellten Promotorregion eine Sequenzidentität von mindestens 70 % - 80 % aufweist.

5. Die Promotorregion gemäß Anspruch 1, die eine Nukleotidsequenz aufweist, die von der in SEQ ID No. 2 dargestellten Sequenz umfasst ist und mindestens die beiden Promotorelemente TATA-Box und CAAT-Box umfasst, und mit der in SEQ ID No. 2 dargestellten Promotorregion Promotorregion unter stringenten Hybridisierungsbedingungen hybridisiert, und mit der in SEQ ID No. 2 dargestellten Promotorregion eine Sequenzidentität von mindestens 70 % - 80 % aufweist.

6. Die Promotorregion gemäß einem der vorangehenden Ansprüche, die eine Nukleotidsequenz aufweist, welche ein RY-Repeat (CATGCATG) zwischen der CAAT-Box und der TATA-Box, und/oder eine E-Box (CACATG) neben der TATA-Box umfasst.

7. Die Promotorregion gemäß einem der vorangehenden Ansprüche, die aus Brassicaceen, insbesondere aus *Brassica napus* stammt.

8. Chimäres Gen, das eine Promotorregion gemäß einem der vorangehenden Ansprüche in operativer Verknüpfung mit einer kodierenden Region umfasst.

9. Nukleinsäuremolekül, das eine Promotorregion oder ein chimäres Gen gemäß einem der vorangehenden Ansprüche umfasst.

10. Transgene Pflanze, die eine Promotorregion, ein chimäres Gen oder ein Nukleinsäuremolekül gemäß einem der vorangehenden Ansprüche enthält, sowie Teile dieser Pflanze und Vermehrungsmaterial dieser Pflanze, wie Protoplasten, Pflanzenzellen, Kalli, Samen, Knollen und Stecklinge, sowie die Nachkommen dieser Pflanze.

11. Die Pflanze gemäß Anspruch 10, bei der es sich um eine Ölsaatpflanze, insbesondere Raps, Rüpsen, Sonnenblume, Sojabohne, Erdnuss, Kokospalme, Ölpalme, Baumwolle oder Lein handelt.

12. Verfahren zur Bereitstellung samenspezifischer Expression einer kodierenden Region in Pflanzensamen, umfassend die Schritte:
a) Herstellung einer Nukleinsäuresequenz, in der eine Promotorregion gemäß einem der Ansprüche 1 bis 7 in operativer Verknüpfung mit einer kodierenden Region vorliegt,
b) Übertragen der Nukleinsäuresequenz aus a) auf pflanzliche Zellen, und
c) Regenerieren vollständig transformierter Pflanzen und, falls erwünscht, Vermehren der Pflanzen.

13. Verwendung einer Promotorregion gemäß einem der Ansprüche 1 bis 7 zur Herstellung von transgenen Pflanzen, Pflanzenzellen, Pflanzenteilen und/oder Pflanzenprodukten mit veränderter Genexpression.

## Claims

1. Isolated promoter region naturally controlling the expression of a plant β-ketoacyl-CoA synthase gene and having a nucleotide sequence which comprises at least the two promoter elements TATA box and CAAT box, wherein this nucleotide sequence is either comprised by the sequence depicted in SEQ ID No. 2 or hybridizes with the promoter region depicted in SEQ ID No. 2 under stringent hybridization conditions, or has a sequence identity to the promoter region depicted in SEQ ID No. 2 of at least 70-80%.

2. The promoter region according to claim 1, having a nucleotide sequence which is comprised by the sequence depicted in SEQ ID No. 2 and comprises at least the two promoter elements TATA box and CAAT box and which hybridizes with the promoter region depicted in SEQ ID No. 2 under stringent hybridization conditions.

3. The promoter region according to claim 1 having a nucleotide sequence which is comprised by the sequence depicted in SEQ ID No. 2 and which comprises at least two promoter elements TATA box and CAAT box and which has a sequence identity of at least 70-80% to the promoter region depicted in SEQ ID No. 2.

4. The promoter region according to claim 1 having a nucleotide sequence which hybridizes with the promoter region depicted in SEQ ID No. 2 under stringent hybridization conditions and which has a sequence identity of at least 70-80% to the promoter region depicted SEQ ID No. 2.

5. The promoter region according to claim 1 having a nucleotide sequence which is comprised by the sequence depicted in SEQ ID No. 2 and which comprises at least the two promoter elements TATA box and CAAT box and which hybridizes with the promoter region depicted in SEQ ID No. 2 under stringent hybridization conditions and which has a sequence identity of at least 70-80% to the promoter region depicted in SEQ ID No. 2.

6. The promoter region according to any of the preceding claims having a nucleotide sequence which comprises an RY repeat (CATGCATG) between the CAAT box and the TATA box and/or an E box (CACATG) next to the TATA box.

7. The promoter region according to any of the preceding claims, which originals from Brassicacee, particularly from *Brassica napus*.

8. Chimeric gene which comprises a promoter region according to any one of the preceding claims being operatively linked with a coding region.

9. Nucleic acid molecule which comprises a promoter region or a chimeric gene according to any one of the preceding claims.

10. Transgenic plant which contains a promoter region, a chimeric gene or a nucleic acid molecule according to any one of the preceding claims, as well as parts of this plant and the propagation material of this plant, such as protoplasts, plant cells, calli, seeds, tubers and cuttings, as well as the progeny of this plant.

11. The plant according to claim 10, which is an oil seed plant, in particular rapeseed, turnip rapeseed, sunflower, soy bean, peanut, coconut palm, oil palm, cotton or flax.

12. Method of providing seed-specific expression of a coding region in plant seeds, comprising the steps of:
a) generation of a nucleic acid sequence in which a promoter region according to any of the claims 1 to 7 is operatively linked with a coding region,
b) transferring the nucleic acid sequence from step a) to plant cells, and
c) regenerating fully transformed plants and, if desired, propagating the plants.

13. Use of a promoter region according to any of claims 1 to 7 for the generation of transgenic plants, plant cells, plant parts and/or plant products with altered gene expression.

## Revendications

1. Région promotrice isolée, qui contrôle nativement l'expression du gène codant pour la β-kétoacyl-CoA-synthase et qui présente une séquence nucléotidique comprenant au moins les éléments promoteurs boîte TATA et boîte CAAT, laquelle séquence nucléotidique étant soit comprise dans la séquence identifiée sous le numéro SEQ.ID N°2, soit s'hybride avec la région promotrice de la séquence identifiée sous le numéro SEQ.ID N°2 dans des conditions d'hybridation très rigoureuses, soit encore présente une identité de séquence avec la région promotrice de la séquence identifiée sous le numéro SEQ.ID N°2 comprise entre au moins 70% à 80%.

2. Région promotrice selon la revendication 1, qui présente une séquence nucléotidique comprise dans la séquence identifiée sous le numéro SEQ.ID N°2, laquelle séquence nucléotidique comprend au moins les éléments promoteurs boîte TATA et boîte CAAT, et s'hybride avec la région promotrice de la séquence identifiée sous le numéro SEQ.ID N°2 dans des conditions d'hybridation très rigoureuses.

3. Région promotrice selon la revendication 1, qui présente une séquence nucléotidique comprise dans la séquence identifiée sous le numéro SEQ.ID N°2, laquelle séquence nucléotidique comprend au moins les éléments promoteurs boîte TATA et boîte CAAT, et présente une identité de séquence avec la région promotrice de la séquence identifiée sous le numéro SEQ.ID N°2 comprise entre au moins 70% à 80%.

4. Région promotrice selon la revendication 1, qui présente une séquence nucléotidique qui s'hybride avec la région promotrice de la séquence identifiée sous le numéro SEQ.ID N°2 dans des conditions d'hybridation très rigoureuses, et qui présente une identité de séquence avec la région promotrice de la séquence identifiée sous le numéro SEQ.ID N°2 comprise entre au moins 70% à 80%.

5. Région promotrice selon la revendication 1, qui présente une séquence nucléotidique comprise dans la séquence identifiée sous le numéro SEQ.ID N°2, laquelle séquence nucléotidique comprend au moins les éléments promoteurs boîte TATA et boîte CAAT, s'hybride avec la région promotrice de la séquence identifiée sous le numéro SEQ.ID N°2 dans des conditions d'hybridation très rigoureuses, et présente une identité de séquence avec la région promotrice de la séquence identifiée sous le numéro SEQ.ID N°2 comprise entre au moins 70% à 80%.

6. Région promotrice selon l'une quelconque des revendications précédentes, qui présente une séquence nucléotidique comprenant une répétition RY (CATGCATG) entre la boîte CAAT et la boîte TATA, et/ou une boîte E (CACATG) à côté de la boîte TATA.

7. Région promotrice selon l'une quelconque des revendications précédentes, qui provient de la famille des Brassica, et plus particulièrement de Brassica napus.

8. Gène chimérique, comprenant une région promotrice selon l'une quelconque des revendications précédentes, lié de manière opérationnelle à une région codante.

9. Molécule d'acide nucléique, comprenant une région promotrice ou un gène chimérique selon l'une quelconque des revendications précédentes.

10. Plante transgénique, contenant une région promotrice, un gène chimérique ou une molécule d'acide nucléique selon l'une quelconque des revendications précédentes, ainsi que des parties de plantes, et matériel de reproduction de ces plantes, tel que protoplastes, cellules végétales, cals, graines, tubercules, boutures, et la progéniture des plantes.

11. Plante selon la revendication 10, selon laquelle la plante est une plante à graine oléagineuse, en particulier le colza, le navet, le tournesol, le soja, la cacahouète, le cocotier, le palmier à huile, le coton ou le lin.

12. Procédé de préparation d'une expression semence-spécifique d'une région codante dans des graines de plante, comprenant les étapes consistant à :
a) fabriquer une séquence d'acide nucléique, dans laquelle une région promotrice selon l'une quelconque des revendications 1 à 7 liée de manière opérationnelle à une région codante est présente;
b) transférer la séquence d'acide nucléique obtenue à l'étape (a) dans des cellules végétales, et
c) régénérer des plantes transformées entières, et le cas échéant, les multiplier.

13. Utilisation d'une région promotrice selon l'une quelconque des revendications 1 à 7 pour la fabrication de plantes, cellules de plantes, parties de plantes et/ou produits de plantes, transgéniques, présentant une expression génétique modifiée.


### SEQUENZPROTOKOLL

(1) ALLGEMEINE ANGABEN:
   (i) ANMELDER:
      (A) NAME: Gesellschaft fuer Erwerb und Verwertung von Schutzrechten - GVS mbH
      (B) STRASSE: Kaufmannstr. 71-73
      (C) ORT: Bonn
      (E) LAND: Deutschland
      (F) POSTLEITZAHL: 53115 Bonn
   (ii) BEZEICHNUNG DER ERFINDUNG: Elongasepromotoren für gewebespezifische Expression von Transgenen in Pflanzen
   (iii) ANZAHL DER SEQUENZEN: 3
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 1785 Basenpaare
      (B) ART: Nucleotid
      (C) STRANGFORM: Doppelstrang
      (D) TOPOLOGIE: linear
   (ii) ART DES MOLEKÜLS: cDNA
   (xi) SEQUENZBESCHREIBUNG: SEQ ID NO: 1:
   AGCGTAACGG ACCACAAAAG AGGATCCATA CAAATACATC TCATCGCTTC CTCTACTATT     60
   CTCCGACACA CACACTGAGC AATGACGTCC ATTAACGTAA AGCTCCTTTA CCATTACGTC    120
   ATAACCAACC TTTTCAACCT TTGCTTCTTT CCGTTAACGG CGATCGTCGC CGGAAAAGCC    180
   TATCGGCTTA CCATAGACGA TCTTCACCAC TTATACTATT CCTATCTCCA ACACAACCTC    240
   ATAACCATCG CTCCACTCTT TGCCTTCACC GTTTTCGGTT CGGTTCTCTA CATCGCAACC    300
   CGGCCCAAAC CGGTTTACCT CGTTGAGTAC TCATGCTACC TTCCACCAAC GCATTGTAGA    360
   TCAAGTATCT CCAAGGTCAT GGATATCTTT TATCAAGTAA GAAAAGCTGA TCCTTCTCGG    420
   AACGGCACGT GCGATGACTC GTCGTGGCTT GACTTCTTGA GGAAGATTCA AGAACGTTCA    480
   GGTCTAGGCG ATGAAACTCA CGGGCCCGAG GGGCTGCTTC AGGTCCCTCC CCGGAAGACT    540
   TTTGCGGCGG CGCGTGAAGA GACGGAGCAA GTTATCATTG GTGCGCTAGA AAATCTATTC    600
   AAGAACACCA ACGTTAACCC TAAAGATATA GGTATACTTG TGGTGAACTC AAGCATGTTT    660
   AATCCAACTC CATCGCTCTC CGCGATGGTC GTTAACACTT TCAAGCTCCG AAGCAACGTA    720
   AGAAGCTTTA ACCTTGGTGG CATGGGTTGT AGTGCCGGCG TTATAGCCAT TGATCTAGCA    780
   AAGGACTTGT TGCATGTCCA TAAAAATACG TATGCTCTTG TGGTGAGCAC AGAGAACATC 840
   ACTTATAACA TTTACGCTGG TGATAATAGG TCCATGATGG TTTCAAATTG CTTGTTCCGT 900
   GTTGGTGGGG CCGCTATTTT GCTCTCCAAC AAGCCTGGAG ATCGTAGACG GTCCAAGTAC 960
   GAGCTAGTTC ACACGGTTCG AACGCATACC GGAGCTGACG ACAAGTCTTT TCGTTGCGTG 1020
   CAACAAGGAG ACGATGAGAA CGGCAAAATC GGAGTGAGTT TGTCCAAGGA CATAACCGAT 1080
   GTTGCTGGTC GAACGGTTAA GAAAAACATA GCAACGTTGG GTCCGTTGAT TCTTCCGTTA 1140
   AGCGAGAAAC TTCTTTTTTT CGTTACCTTC ATGGGCAAGA AACTTTTCAA AGATAAAATC 1200
   AAACATTACT ACGTCCCGGA TTTCAAACTT GCTATTGACC ATTTTTGTAT ACATGCCGGA 1260
   GGCAGAGCCG TGATTGATGT GCTAGAGAAG AACCTAGCCC TAGCACCGAT CGATGTAGAG 1320
   GCATCAAGAT CAACGTTACA TAGATTTGGA AACACTTCAT CTAGCTCAAT ATGGTATGAG 1380
   TTGGCATACA TAGAAGCAAA AGGAAGGATG AAGAAAGGTA ATAAAGTTTG GCAGATTGCT 1440
   TTAGGGTCAG GCTTTAAGTG TAACAGTGCA GTTTGGGTGG CTCTAAACAA TGTCAAAGCT 1500
   TCGACAAATA GTCCTTGGGA ACACTGCATC GACAGATACC CGGTCAAAAT TGATTCTGAT 1560
   TCAGGTAAGT CAGAGACTCG TGTCCAAAAC GGTCGGTCCT AATAAATGAT GTTTGCTCTC 1620
   TTTCGTTTCT TTTTATTTGT TATAATAATT TGATGGCTAC GATGTTTCTC TTGTTTGTTA 1680
   TGAATAAAGA ATGCAATGGT GTTCTAGTAT TTGATTGTTT TACATGTATG TATCTCTTAT 1740
   TTACATGAAA TTTTTAAACG CCTAGGAAAA AAAAAAAAAA AAAAA 1785
(2) ANGABEN ZU SEQ ID NO: 2:
   (i) SEQUENZKENNZEICHEN:
      (A) LÄNGE: 1468 Basenpaare
      (B) ART: Nucleotid
      (C) STRANGFORM: Doppelstrang
      (D) TOPOLOGIE: linear
   (ii) ART DES MOLEKÜLS: Genom-DNA
   (xi) SEQUENZBESCHREIBUNG: SEQ ID NO: 2:
   AAGCTTTACA ACGATACACA AAACTTATAA CCGTAATCAC CATTCATTAA CTTAACTACT     60
   ATCACATGCA TTCATGAATT GAAACGAGAA GGATGTAAAT AGTTGGGAAG TTATCTCCAC    120
   GTTGAAGAGA TCGTTAGCGA GAGCTGAAAG ACCGAGGGAG GAGACGCCGT CAACACGGAC    180
   AGAGTCGTCG ACCCTCACAT GAAGTAGGAG GAATCTCCGT GAGGAGCCAG AGAGACGTCT    240
   TTGGTCTTCG GTTTCGATCC TTGATCTGAC GGAGAAGACG AGAGAAGTGC GACTGGACTC    300
   CGTGAGGACC AACAGAGTCG TCCTCGGTTT CGATCGTCGG TATTGGTGGA GAAGGCGGAG    360
   GAATCTCCGT GACGAGCCAG AGAGATGTCG TCGGTCTTCG GTTTCGATCC TTGATCTGAC    420
   GGAGAAGACG AGAGAAGTGC GACGAGACTC CGTGAGGACC AACAGAGTTG TCCTCGGTTT    480
   CGATCGTCGG TTTCGGCGGA GAAGGCGGAG GAATCTCCGT GAGGAGCCAG AGAGACGTCG    540
   TTGGTCTTCG GTTTCGATCC TTGATCTGAT GGAGAAGACG AGACAAGTGG GACGAGACTC    600
   AACGACGGAG TCAGAGACGT CGTCGGTCTT CGGTTTCGGC CGAGAAGGCG GAGTCGGTCT    660
   TCGGTTTCGG CCGAGAAGGC GGAGGAGACG TCTTCGATTT GGGTCTCTCC TCTTGACGAA    720
   GAAAACAAAG AACACGAGAA ATAATGAGAA AGAGAACAAA AGAAAAAAAA ATAAAAATAA    780
   AAATAAAATT TGGTCCTCTT ATGTGGTGAC ACGTGGTTTG AAACCCACCA AATAATCGAT    840
   CACAAAAAAC CTAAGTTAAG GATCGGTAAT AACCTTTCTA ATTAATTTTG ATTTAATTAA    900
   ATCACTCTTT TTATTTATAA ACCCCACTAA ATTATGCGAT ATTGATTGTC TAAGTACAAA    960
   AATTCTCTCG AATTCAATAC ACATGTTTCA TATATTTAGC CCTGTTCATT TAATATTACT   1020
   AGCGCATTTT TAATTTAAAA TTTTGTAAAC TTTTTTGGTC AAAGAACATT TTTTTAATTA   1080
   GAGACAGAAA TCTAGACTCT TTATTTGGAA TAATAGTAAT AAAGATATAT TAGGCAATGA   1140
   GTTTATGATG TTATGTTTAT ATAGTTTATT TCATTTTAAA TTGAAAAGCA TTATTTTTAT   1200
   CGAAATGAAT CTAGTATACA ATCAATATTT ATGTTTTTTC ATCAGATACT TTCCTATTTT   1260
   TTGGCACCTT TCATCGGACT ACTGATTTAT TTCAATGTGT ATGCATGCAT GAGCATGAGT   1320
   ATACACATGT CTTTTAAAAT GCATGTAAAG CGTAACGGAC CACAAAAGAG GATCCATACA   1380
   AATACATCTC ATCGCTTCCT CTACTATTCT CCGACACACA CACTGAGCAA TGACGTCCAT   1440
   TAACGTAAAG CTCCTTTACC ATTACGTC                                      1468
(2) ANGABEN ZU SEQ ID NO: 3:
   (i) SEQUENZKENNZEICHEN:
      (A) LÄNGE: 1785 Basenpaare
      (B) ART: Nucleotid
      (C) STRANGFORM: Doppelstrang
      (D) TOPOLOGIE: linear
   (ii) ART DES MOLEKÜLS: cDNA
   (ix) MERKMAL:
      (A) NAME/SCHLÜSSEL: CDS
      (B) LAGE:82..1599
   (xi) SEQUENZBESCHREIBUNG: SEQ ID NO: 3: